# EUROPÄISCHE PATENTSCHRIFT

(11) **EP 1 911 748 B1**
(45) Veröffentlichungstag und Bekanntmachung des Hinweises auf die Patenterteilung: **07.04.2010**
(21) Anmeldenummer: 07025075.8
(22) Anmeldetag: 28.09.2000
(51) Int. Cl.: C07D 251/16, C07C 303/38, C07C 311/65

(54) **Verfahren zur Herstellung substituierter Phenylsufonylharnstoffe aus Sulfohalogeniden**
Method for producing substituted phenylsulfonyl ureas from sulfohalogenides
Procédé de production de urées de phenylsulfonyl substituées issus de sulfo-halogenures

(30) Priorität: 28.09.1999 DE 19946341
(43) Veröffentlichungstag der Anmeldung: 16.04.2008
(62) Teilanmeldung aus: 00971292.8
(73) Patentinhaber: Bayer CropScience AG, 40789 Monheim (DE)
(72) Erfinder: Vermehren, Jan, Dr., 65510 Idstein (DE); Schmidt, Ernst, Dr., 41569 Rommerskirchen (DE); Ford, Mark James, Dr., 61389 Schmitten-Oberreifenberg (DE); Foster, Richard, W.G. Dr., Huntingdon, Cambridgeshire PE28 2BN (GB); Bourne, Ian, A. Dr., Cambridge CB1 4YP (GB)

(56) Entgegenhaltungen:
- EP-A- 0 046 626
- WO-A-92/13845
- WO-A-96/06826
- US-A- 2 852 557
- US-A- 4 238 621
- US-A- 4 849 010

## Beschreibung

Die Erfindung betrifft das technische Gebiet der chemischen Verfahren zur Herstellung von Verbindungen aus der Reihe herbizider Phenylsulfonylharnstoffe und deren Zwischenprodukte.

Eine Reihe von substituierten Phenylsulfonylharnstoffen sind als Herbizide und Pflanzenwachstumsregulatoren beschrieben worden. Innerhalb der Gruppe der Phenylsulfonylharnstoffe werfen solche mit einer Carboxygruppe oder einer Carbonsäurederivatgruppe am Phenylring besondere synthetische Fragen auf. Von Interesse sind die aus EP-A-007687 oder WO-A-92/13845 bekannten Verbindungen der Formel (I) und deren Salze, 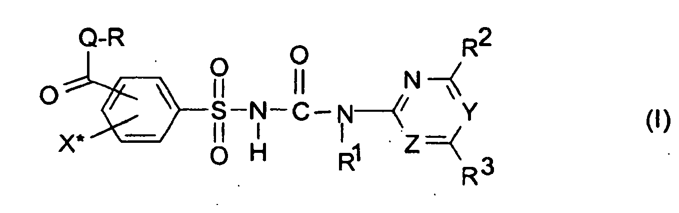 worin
- Q: Sauerstoff, Schwefel oder -N(R⁴)-,
- X*: Halogen, insbesondere Iod,
- Y,Z: unabhängig voneinander CH oder N, wobei Y und Z nicht gleichzeitig CH sind,
- R: Wasserstoff, (C₁-C₁₂)-Alkyl; (C₂-C₁₀)-Alkenyl; (C₂-C₁₀)-Alkinyl; (C₁-C₆)-Alkyl, das ein- bis vierfach durch Reste aus der Gruppe Halogen, (C₁-C₄)-Alkoxy, (C₁-C₄)-Alkylthio, CN, [(C₁-C₄)-Alkoxy]-carbonyl und (C₂-C₆)-Alkenyl substituiert ist; oder (C₃-C₈)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist; (C₅-C₈)-Cycloalkenyl; Phenyl-(C₁-C₄)-alkyl, das im Phenylrest unsubstituiert oder durch einen oder mehrere Reste aus der Gruppe Halogen, (C₁-C₄)-Alkyl, (C₁-C₄)-Alkoxy, (C₁-C₄)-Haloalkyl, (C₁-C₄)-Alkylthio, [(C₁-C₄)-Alkoxy]-carbonyl, [(C₁-C₄)-Alkyl]-carbonyloxy, Carbonamid, [(C₁-C₄)-Alkyl]-carbonylamino, [(C₁-C₄)-Alkyl]-aminocarbonyl, Di-[(C₁-C₄)-alkyl]-aminocarbonyl und Nitro substituiert ist; oder einen Rest der Formeln A-1 bis A-10 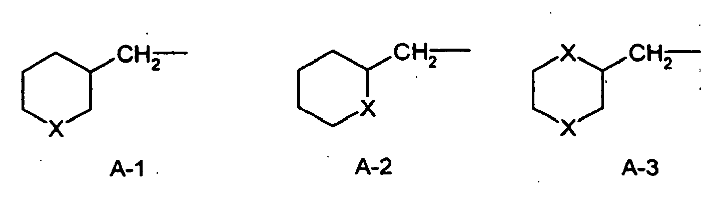 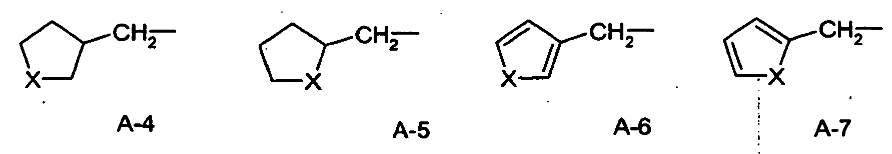 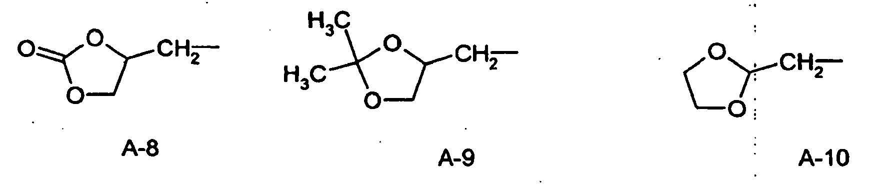
worin
- X: O, S, S(O) oder SO₂;
- R¹: Wasserstoff oder (C₁-C₃)-Alkyl;
- R²: Wasserstoff, Halogen, (C₁-C₃)-Alkyl oder (C₁-C₃)-Alkoxy, wobei jeder beiden letztgenannten Reste unsubstituiert oder ein- oder mehrfach durch Halogen oder (C₁-C₃)-Alkoxy substituiert ist;
- R³: Wasserstoff, Halogen, (C₁-C₃)-Alkyl, (C₁-C₃)-Alkoxy oder (C₁-C₃)-Alkylthio, wobei jeder der letztgenannten drei Reste unsubstituiert oder ein- oder mehrfach durch Halogen oder ein- oder zweifach durch (C₁-C₃)-Alkoxy oder (C₁-C₃)-Alkylthio substituiert ist; oder einen Rest der Formel NR⁵R⁶, (C₃-C₆)-Cycloalkyl, (C₂-C₄)-Alkenyl, (C₂-C₄)-Alkinyl, (C₃-C₄)-Alkenyloxy oder (C₃-C₄)-Alkinyloxy;
- R⁴: Wasserstoff, (C₁-C₄)-Alkyl oder (C₁-C₄)-Alkoxy und
- R⁵ und R⁶: unabhängig voneinander Wasserstoff, (C₁-C₄)-Alkyl, (C₃-C₄)-Alkenyl, (C₁-C₄)-Haloalkyl oder (C₁-C₄)-Alkoxy bedeuten.

Die Salze der Verbindungen (I) sind vorzugsweise Verbindungen, in denen das Wasserstoffatom in der SO₂NH-Grupp des Sulfonylharnstoffs durch ein Kation ersetzt ist, vorzugsweise ein physiologisch verträgliches und im Pflanzenschutz einsetzbares Kation, insbesondere ein Alkalimetall- oder Erdalkalimetallkation oder ein gegebenenfalls substituiertes Ammoniumion, inklusive quartäre Ammoniumionen. Beispiele für Kationen sind das Natrium-, Kalium- und Ammoniumion.

Salze der Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, gebildet werden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Salze können ebenfalls dadurch gebildet werden, daß bei geeigneter funktionellar Gruppe, wie z.B. der Carboxygruppe, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quaternäre Ammoniumsalze.
Von besonderem Interesse sind Verbindungen der Formel (I) oder deren Salze, worin die Gruppe der Formel -CO-Q-R in Orthostellung zur Sulfonylgruppe des Sulfonylharnstoffs (I) steht. Bevorzugt sind Verbindungen (I) oder deren Salze, worin Q = Sauerstoffatom, X* = Wasserstoff oder Halogen, vorzugsweise Iod, R = (C₁-C₄)-Alkyl; (C₂-C₄)-Alkenyl; (C₂-C₄)-Alkinyl; (C₁-C₄)-Haloalkyl, oder (C₁-C₄)-Alkoxy(C₁-C₄)-alkyl, vorzugsweise Methyl oder Ethyl, insbesondere Methyl bedeuten. Weiter bevorzugt sind Verbindungen (I) und deren Salze, worin die Gruppe der Formel -CO-Q-R in Orthostellung zur Sulfonylgruppe des Sulfonylharnstoffs, X* = Halogen, vorzugsweise Iod, und X* in Parastellung zur Gruppe der Formel -CO-Q-R steht.

Gemäß WO-A-92/13845 ist bekannt, die Verbindungen (I) oder deren Salze aus entsprechend substituierten Phenylsulfonylisocyanaten durch Umsetzung mit heterocyclischen Aminen, beispielsweise 2-Amino-4-methoxy-6-methyl-1,3,5-triazin oder 2-Amino-4,6-dimethoxy-pyrimidin, herzustellen. Die Phenylsulfonylisocyanate andererseits sind beispielsweise aus den entsprechenden Phenylsulfochloriden nach Ammonolyse zu Sulfonamiden und Umsetzung der Sulfonamide zu den Sulfonylisocyanaten nach Standardmethoden zugänglich.

Zur Durchführung der Ammonolyse kann beispielsweise das Sulfonylchlorid in einem organischen Lösungsmittel wie Tetrahydrofuran (THF) gelöst und Ammoniakgas eingeleitet werden (vgl. WO-A-92/13845).
Als Standardmethode zur Herstellung des substituierten Phenylsulfonylisocyanats aus dem erhaltenen Phenylsulfonamid kommt die sequentielle Behandlung des Phenylsulfonamids mit Thionylchlorid und Phosgen in Frage, gegebenenfalls in Gegenwart katalytischer Mengen einer Base wie Pyridin; vgl. WO-A-92/13845, Beispiel 11. Alternativ kann das Phenylsulfonamid auch mit einem Alkylisocyanat unter Verwendung sterisch gehinderter Aminbasen als Katalysatoren zu dem entsprechend substituierten N-Alkyl-N'-phenylsulfonyl-harnstoff umgesetzt werden, der dann zum substituierten Phenylsulfonylisocyanat phosgeniert wird; vgl. auch WO-A-92/13845, Beispiel 12. Vergleichbare Umsetzungen sind auch für strukturell andere Phenyl- oder Heteroarylsulfonamide zur Herstellung der Sulfonylisocyanate beschrieben; siehe z. B. US-A-4,647303, EP-A-4602942, EP-A-0184385, EP-A-0727423.

Bekannt ist auch die direkte Umsetzung von Aryl- oder Heteroarylsulfonamiden mit Phosgen in Gegenwart von Alkyl- oder Cycloalkylisocyanaten und gegebenenfalls in Gegenwart von sterisch gehinderte Aminbasen (US-A-4,647303, EP-A-0584043, EP-A-0030138, EP-A-0184385).

WO-A-96/06826 beschreibt ein Verfahren zur Phosgenierung von Phenylsulfonamiden, von denen einige auch orthoständige Carbalkoxygruppen am Phenylring aufweisen, zum entsprechend substituierten Phenylsulfonylisocyanat durch Umsetzung mit Phosgen in Gegenwart eines Katalysators, der aus Butylisocyanat und tertiären Aminen wie DABCO und/oder aus substituierten Sulfonylisocyanaten besteht. In einem Beispiel wird als Katalysator n-Butylisocyanat in Kombination mit einer geringen Menge des Endprodukts (als ein substituiertes Sulfonylisocyanat) aus einem früheren Ansatz als Ko-Katalysator verwendet. Bei der dort bevorzugten Verfahrensweise wird das Phosgen ständig im Überschuss bezogen auf das eingesetzte Sulfonamid gehalten.

Die genannten bekannten Verfahren zur Herstellung der Phenylsulfonamide, der Phenylsulfonylisocyanate und der Verbindungen der Formel (I) sind jedoch hinsichtlich der chemischen Ausbeuten, der Raum-Zeit-Ausbeuten und/oder des apparatetechnischen Aufwands unbefriedigend. Beispielsweise werden im Falle von Phenylsulfonylharnstoffen mit Carbalkoxygruppen in 2-Stellung am Phenylrest bei der Ammonolyse Nebenprodukte wie das entsprechende Saccharinderivat gebildet.

Bei der Phosgenierung des entsprechend substituierten N-Alkyl-N'-phenylsulfonyl-harnstoffs treten häufig signifikante Mengen an substituiertem N,N'-Bis-(phenylsulfonyl)-harnstoff als Nebenprodukt auf. Diese Phosgenierungsmethode benötigt außerdem vergleichsweise lange Reaktionszeiten, insbesondere für die Anfangsphase (Einsetzen der Phosgenierung). Die Herstellung des N-Alkyl-N'-phenylsulfonyl-harnstoffs erfordert nach der bekannten Weise sterisch gehinderte Aminbasen, deren Einsatz und Abtrennung mit technischem und wirtschaftlichem Aufwand verbunden ist.
Für die herzustellenden Phenylsulfonylisocyanate, die im Phenylrest durch X* = Halogen, vorzugsweise Iod, substituiert sind, sind bislang nur wenige Methoden bekannt (vgl. WO-A-92/13845). Welche Methode für derartige Verbindungen eine weitere Verbesserung der Herstellung ermöglichen könnte, ist bisher nicht bekannt gewesen. Vor allem wegen der strukturellen Besonderheiten wie z. B. der sterischen Hinderung und Reaktivität am substituierten Phenylrest dieser Verbindungen lassen sich nur wenige Ergebnisse von Verfahren mit strukturell anderen Aryl- oder Heteroarylsulfonamiden analog erwarten.

Die Umsetzung der erhaltenen Sulfonylisocyanate mit heterocyclischen Aminen zu Verbindungen der Formel (I) kann gemäß bekannten Methoden auf verschiedene Weise erfolgen.

Standardmäßig können die Umsetzungen von Phenylsulfonylisocyanaten mit Aminopyrimidinen oder Aminotriazinen in einem organischen Lösungsmittel erfolgen. Als Lösungsmittel werden dabei polare aprotische Lösungsmittel wie THF und Acetonitril empfohlen (siehe z. B. EP-A-0030138, Seiten 14-15).

Aus SU 1233456 (1996) ist die Verwendung von N-Methylpyrrolidon (NMP) in einem aprotischen organischen Lösungsmittel zur Kupplung von Aminotriazinen mit Arylsulfonylisocyanaten bekannt. Bei einem Anteil von 1 bis 20 % NMP im Lösungsmittel Xylol werden Ausbeuten von etwa 80 bis 82 % innerhalb von 2 bis 4 Stunden Reaktionszeit erreicht. Die Ausbeuten sind für die Durchführung im technischen Maßstab nicht befriedigend.

Nach US-A-04647303 und US-A-04602942 wird zur Beschleunigung der Kupplungsreaktion DABCO eingesetzt. Die Verwendung von 1,4-Diazabicyclo[2.2.2]octan (DABCO) ist im technischen Maßstab nachteilig, weil es zusätzlich eingesetzt und nachher mit besonderem Aufwand wieder abgetrennt werden muß.

Es besteht deshalb die Aufgabe ein alternatives Verfahren bereitzustellen, das im Vergleich zu den bekannten Verfahren hinsichtlich eines Aspekts, vorzugsweise mehrerer Aspekte vorteilhaft durchzuführen ist.

Gegenstand der Erfindung ist ein Verfahren zur Herstellung der genannten Phenylsulfonylharnstoffe der Formel (I) und deren Salzen,
dadurch gekennzeichnet, daß man
a) eine Verbindung der Formel (II) 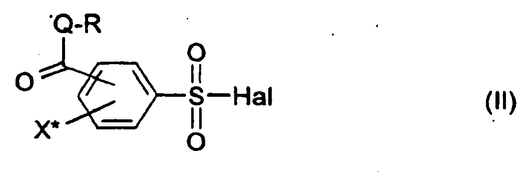 worin Hal ein Halogenatom, vorzugsweise Chlor, Brom oder Iod, insbesondere Chlor bedeutet und R, Q und X* wie in Formel (I) definiert sind, durch Ammonolyse mit Ammoniak zur Verbindung der Formel (III) umsetzt, 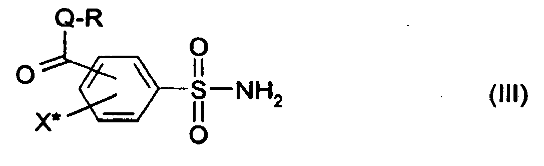 worin R, Q und X* wie in Formel (I) definiert sind,
   vorzugsweise
   (a1) die Umsetzung in einem organischen Lösungsmittelgemisch enthaltend
      (1) einen oder mehrere gegebenenfalls halogenierte aromatische Kohlenwasserstoffe, beispielsweise Xylol, Toluol, Chlorbenzol oder Dichlorbenzol, [Lösungsmittel (1)] und
      (2) ein oder mehrere polare aprotische Lösungsmittel [Lösungsmittel (2)], vorzugsweise aus der Gruppe enthaltend Nitrile, wie Acetonitril, und Ester, vorzugsweise (C₁-C₂)-Alkancarbonsäure-(C₁-C₆)-alkylester wie Essigsäureethylester, Essigsäureisopropylester, Essigsäure-n-butylester, Essigsäure-n-pentylester und andere Essigsäureamylester, und Ether, wie Tetrahydrofuran (THF) oder 1,2-Dimethoxyethan (DME), Amide, wie Dimethylformamid (DMF), Ketone, wie Aceton, Methylethylketon oder Methylisobutylketon (MIBK), und Gemische aus zwei oder mehreren der polaren Lösungsmittel,
      im Gewichtsverhältnis Lösungsmittel (1):Lösungsmittel (2) von 20:1 bis 1:1, vorzugsweise von 10:1 bis 1,4:1 durchführt, insbesondere die genannte bevorzugte Umsetzung unter Zugabe einer Lösung der Verbindung (II) in Lösungsmittel (1) oder einem Lösungsmittelgemisch der Lösungsmittel (1) und (2) zur konzentrierten oder gesättigten Lösung von Ammoniak im Lösungsmittel (2) durchführt,
(b1) die erhaltene Verbindung (III) mit oder ohne Zwischenisolierung mit Phosgen zum Phenylsulfonylisocyanat der Formel (IV) umsetzt, 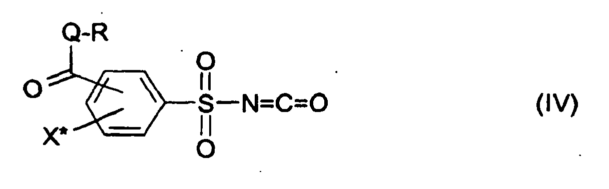 worin R, Q und X* wie in Formel (I) definiert sind, und dabei die Umsetzung mit Phosgen in einem organischen Lösungsmittel in Gegenwart eines Isocyanats der Formel R'-NCO als Katalysator, wobei R' ein Kohlenwasserstoffrest ist, der unsubstituiert oder substituiert ist und inklusive vorzugsweise 1 bis 20 C-Atome, insbesondere 1 bis 16 C-Atome aufweist, oder eines Gemisches mehrerer dieser Isocyanate als Katalysator, vorzugsweise eines oder mehrerer Isocyanate aus der Gruppe N-Alkylisocyanate, N-Cycloalkylisocyanate und N-Arylisocyanate, beispielsweise eines N-(C₁-C₁₂)Alkylisocyanats aus der Gruppe enthaltend Methylisocyanat, Ethylisocyanat, n-Propylisocyanat, Isopropylisocyanat, n-, i-, sec- oder tert-Butylisocyanat, Pentylisocyanat, Hexylisocyanat, Heptylisocyanat, Cyclohexylisocyanat und Phenylisocyanat, insbesondere n-Butylisdcyanat oder Cyclohexylisocyanat,
   ohne Zugabe einer Aminbase oder anderen Base als Ko-katalysator durchführt, insbesondere die Umsetzung unter Vorlage einer Menge des Sulfonylisocyanats der Formel (IV) beginnt,

(c) die erhaltenen Verbindung (IV) mit oder ohne Zwischenisolierung in einem organischen Lösungsmittel mit einem Amin der Formel (V) 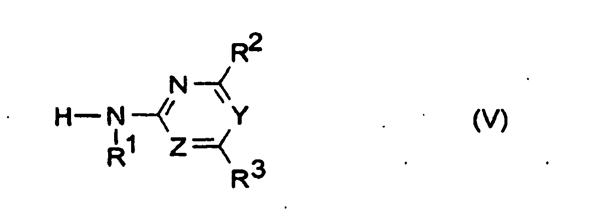 worin R¹, R², R³, Y und Z wie in Formel (I) definiert sind,
   zum Sulfonylharnstoff der Formel (I) oder dessen Salzen umsetzt, vorzugsweise
   (c1) die Umsetzung in einem Lösungsmittelgemisch aus einem gegebenenfalls halogenierten aromatischen Kohlenwasserstoff mit einem Siedepunkt von mehr als 110 °C und einem polaren aprotischen Lösungsmittel, vorzugsweise Xylol/Essigsäureethylester oder Xylol/Acetonitril durchführt,
wobei im Verfahren mindestens einer der bevorzugten Teilschritte (a1), (b1) und (c1) durchgeführt wird.

Gegenstand der Erfindung sind auch die erfindungsgemäßen Teilschritte (a1) und (c1) des Verfahrens sowie der Teilschritt (b1), vorzugsweise für den Fall X* lod, und deren mehrstufig kombinierte Verfahrensweise ober entsprechende Eintopfverfahren.

In den Definitionen der Formeln (I) bis (V) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein.

Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, vorzugsweise 3 bis 6 C-Atomen, z.B. Cyclopropyl, Cyclobutyl, Cyclopentyl oder Cyclohexyl.

Halogen bedeutet beispielsweise Fluor, Chlor, Brom oder Iod. In Restedefinitionen wird mit "Halogen" ein Halogenrest, d. h. ein Halogenatom bezeichnet. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein carbozyklisches aromatisches System, beispielsweise ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein Kohlenwasserstoffrest [siehe z. B. die Definition von R' in Stufe (b1)] enthält ausschließlich C-Atome und H-Atome und kann geradkettig, verzweigt oder cyclisch, gesättigt, ungesättigt oder aromatisch sein oder kann eine Kombination gleicher oder verschiedener der weiter oben genannten Kohlenwasserstoffreste enthalten. Beispielsweise werden von "Kohlenwasserstoffrest" die Reste Alkyl, Alkenyl, Alkinyl, Cycloalkyl, Cycloalkenyl, Cycloalkyl-alkyl, Cycloalkenyl-alkyl, Aryl wie Phenyl oder Naphthyl, Benzyl, Phenethyl etc. umfasst. Ein Kohlenwasserstoffrest enthält vorzugsweise 1 bis 30 C-Atome, insbesondere 1 bis 24 C-Atome, sofern nichts Anderes definiert ist.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Kohlenwasserstoffrest, z. B. substituierter Alkyl-, Alkenyl-, Alkinyl-, Aryl-, Phenyl-, Benzyl-rest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Alkylsulfinyl und Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten.
Bevorzugt sind Substituenten aus der Gruppe Halogen, Alkoxy, Alkylthio, Hydroxy, Amino, Nitro, Cyano, Mono- und Dialkylamino, im Falle cyclischer Reste, auch Alkyl und Haloalkyl;
im Begriff "substituierte Reste" wie substituierte Kohlenwasserstoffreste wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.
Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.
Bei den genannten Substituenten ist jeweils die Anzahl der C-Atome bevorzugt, wie sie weiter oben für Reste mit Kohlenwasserstoffanteilen als bevorzugt genannt sind.

Die Verbindungen der Formel (II) sind bekannt oder können nach bekannten Methoden hergestellt werden. Diesbezüglich wird insbesondere auf den Inhalt der

Druckschriften EP-A-007687 und WO-A-92/13845 und dort zitierte Literatur Bezug genommen. Der Inhalt der WO-A-92/13845 soll im übrigen hinsichtlich der Verfahrensvorschriften und der dort genannten bevorzugten Verbindungen der Formel (I) und deren Vorprodukten Bestandteil der vorliegenden Beschreibung und Erfindung sein.
Besonders bevorzugt sind für das Herstellungsverfahren Verbindungen der Formel (II), worin Q = Sauerstoffatom, X* = Wasserstoff oder Halogen, vorzugsweise Iod,
R = (C₁-C₄)-Alkyl; (C₂-C₄)-Alkenyl; (C₂-C₄)-Alkinyl; (C₁-C₄)-Haloalkyl, oder (C₁-C₄)-Alkoxy(C₁-C₄)-alkyl, vorzugsweise Methyl oder Ethyl, insbesondere Methyl bedeutet und Hal = Chlor bedeutet.

Die Ammonolyse des Sulfochlorids (II) kann nach den üblichen Methoden für Ammonolysen durchgeführt werden, beispielsweise wie sie Handbüchern der Chemie oder in WO-A-92/13845 beschrieben sind. Eine übliche Methode beinhaltet die Anwendung von Ammoniakgas, das in eine Lösung des Sulfochlorids (II) in einem organischen Lösungsmittel eingeleitet wird. Als organische Lösungsmittel eignen sich beispielsweise polare aprotische Lösungsmittel wie THF oder Aceton. Die Umsetzung gelingt in der Regel bereits bei Raumtemperatur. Zur Aufarbeitung muß entstandenes Ammoniumchlorid vom Produkt getrennt werden, was auf verschiedene und dem Fachmann geläufige Weise möglich ist.

Das in WO-A-92/13845 beschriebene Verfahren zur Ammonolyse verwendet THF als alleiniges Lösungsmittel. Das Verfahren ist bei größeren Ansatzmengen und im technischen Maß nicht befriedigend durchführbar. So werden dabei in der Regel Ausbeuten von weniger als 80% d. Th. erreicht, was vor allem auf der Entstehung von Nebenprodukten zurückzuführen ist, z. B. von Sacharin(derivaten) durch intramolekulare Reaktion.

Ein Gegenstand der Erfindung besteht in einem verbesserten Verfahren zur Ammonolyse, speziell das Verfahren nach der genannten bevorzugten Variante (a1). Erfindungsgemäß wird die Umsetzung in einem Lösungsmittelgemisch aus Lösungsmittel (1) und Lösungsmittel (2) im Gewichtsverhältnis Lösungsmittel (1):Lösungsmittel (2) von 20:1 bis 1:1, vorzugsweise von 10:1 bis 1,4:1 durchgeführt. Dies entspricht einem Gemisch aus einem gegebenenfalls halogenierten, aromatischen Kohlenwasserstoff , dem 5 bis 100 Gew.-%, vorzugsweise 10-71 Gewichtsprozent des polaren Lösungsmittels (2) zugesetzt ist. Erfindungsgemäß ist auch das Verfahren, bei dem der Reaktionsansatz am Anfang mit einem Lösungsmittel (1) oder (2) oder einem Lösungsmittelgemisch beginnt, bei dem der Anteil des Lösungsmittels (1) oder (2) außerhalb des erfindungsgemäßen Gewichtsverhältnisses ist, sofern die Zugabe des unpolaren bzw. polaren Lösungsmittels im Laufe der Reaktion ein erfindungsgemäßes Mengenverhältnis ergibt.

Besonders bevorzugt ist Xylol als Lösungsmittel (1). Bevorzugt als polare Lösungsmittel (2) sind Acetonitril, Essigsäureethylester, Essigsäureisopropylester, ionsäure, Essigsäure-n-butylester, Tetrahydrofuran, 1,2-Dimethoxyethan (DME), Dimethylformamid, Methylethylketon, Methylisobutylketon und Gemische aus zwei oder mehreren der polaren Lösungsmittel, insbesondere Acetonitril, oder Essigsäureethylester.

Die Verbindung (II) kann dabei beispielsweise in dem Gemisch der Lösungsmittel (1) und (2) vorgelegt und Ammoniak als Lösung oder als Gas zugeführt werden. Alternativ kann die Verbindung (II) in einem Lösungsmittel (1) vorgelegt und Ammoniak als Lösung im Lösungsmittel (2) zugegeben werden. Weiterhin kann Ammoniak in Lösung vorgelegt und eine Lösung der Verbindung (II) zugefügt werden. Sinnvoll ist dabei sowohl eine kontinuierliche Zugabe der einen Reaktionskomponente oder eine portionsweise Zugabe. Ebenso können beide Reaktionskomponenten parallel kontinuierlich oder portionsweise in das Reaktionsgefäß gegeben werden.

Bevorzugt wird die Umsetzung unter Zugabe einer Lösung der Verbindung (II) im Lösungsmittel (1) oder einem Lösungsmittelgemisch der Lösungsmittel (1) und (2)
zur konzentrierten oder gesättigten Lösung von Ammoniak im Lösungsmittel (2) durchführt. Insbesondere bevorzugt wird dabei der Verbrauch an Ammoniak während der Reaktion durch Einleiten von Ammoniakgas ausgeglichen, so dass die Reaktionslösung stets mit Ammoniak gesättigt oder hochkonzentriert ist.

Die Reaktionstemperatur für die Ammonolyse liegt beispielsweise im Bereich von -20 °C bis zum Siedepunkt des jeweiligen Lösungsmittels bzw. Lösungsmittelgemisches, vorzugsweise im Bereich von 0 °C bis +80 °C, insbesondere im Bereich von 20 °C bis 60 °C.
Die Reaktion kann bei Normaldruck aber auch bei Unter- oder Überdruck durchgeführt werden.

Die nach der Ammonolyse erhaltene Verbindung (III) (Phenylsulfonamid) kann mit oder ohne Zwischenisolierung mit Phosgen zum Phenylsulfonylisocyanat der Formel (IV) umgesetzt werden. Für die Bedingungen der Umsetzung kommen übliche Bedingungen zur Herstellung von Sulfonylisocyanaten aus Sulfonamiden in Frage.

Beispielsweise die anfangs erwähnte, aus WO-A-92/13845 bekannte sequentielle Behandlung des Phenylsulfonamids mit Thionylchlorid und Phosgen in Gegenwart katalytischer Mengen einer Base wie Pyridin.
Alternativ kann gemäß bekannter Verfahrensweise das Phenylsulfonamid mit einem Alkylisocyanat in Gegenwart einer sterisch gehinderter organischen Base wie 1,8-Diazabicyclo[5.4.0]undec-7-en (DBU), 1,4-Diazabicyclo[2.2.2]octan (DABCO) oder 1,5-Diazabicyclo[4.3.0]non-5-en (DBN) als Katalysatoren zu einem N-Alkyl-N'-phenylsulfonyl-harnstoff umgesetzt werden, der dann mit Phosgen zum substituierten Phenylsulfonylisocyanat reagiert; vgl. WO-A-92/13845, Beispiel 12. Die analoge direkte Umsetzung im Eintopfverfahren ist in EP-A-0030138 beschrieben.

Für die Herstellung der Phenylsulfonylisocyanate der Formel (IV), vorzugsweise solcher Verbindungen (IV), worin X* Iod bedeutet, vorzugsweise solcher Verbindungen (IV), worin die Gruppe -CO-Q-R in Orthostellung zur Sulfonylisocyanatgruppe steht und
insbesondere dabei solche, worin die Gruppe X* in Parastellung zur Gruppe -CO-Q-R steht, ergeben die genannten bekannten Verfahrensalternativen in der Regel jedoch nicht die gewünschten Ausbeuten und Reinheiten.

Erfindungsgemäß lassen sich Nachteile der genannten bekannten Methoden bei der Herstellung der bevorzugten Verbindungen (IV) vermeiden, wenn man die entsprechend substituierten Phenylsulfonamide der Formel (III) gemäß der Verfahrensweise (b1) mit Phosgen in einem organischen Lösungsmittel in Gegenwart eines Isocyanats der genannten Formel R'-NCO als Katalysator ohne Zugabe einer Aminbase oder anderen Base als Ko-katalysator zu den entsprechenden Sulfonylisocyanaten der Formel (IV) umsetzt.

Dabei ist das Verfahren ohne Zusatz von sterisch gehinderten Aminbasen bevorzugt, weil die Aminbasen sonst mit besonderem Aufwand aus dem Produkt entfernt werden müssen.

Als organische Lösungsmittel eignen sich für die Phosgenierung beispielsweise unter den Reaktionsbedingungen inerte, aprotische organische Lösungsmittel vorzugsweise
- ein gegebenenfalls halogenierter aromatischer Kohlenwasserstoff, beispielsweise Toluol, Xylol, Mesitylen, Chlorbenzol, Chlortoluol oder Dichlorbenzol,
- ein polares aprotisches Lösungsmittel aus der Gruppe der Alkancarbonsäurealkylester, vorzugsweise aus der Gruppe der (C₁-C₂)-Alkancarbonsäure-(C₁-C₆)-alkylester wie Essigsäureethylester, Essigsäureisopropylester, Essigsäure-n-butylester, Essigsäure-n-pentylester und andere Essigsäureamylester,
und Gemische aus zwei oder mehreren der genannten Lösungsmittel.
Vorzugsweise werden in der Stufe (a) und (b) des Gesamtverfahrens die gleichen Lösungsmittel oder Lösungsmittelgemische verwendet.

Das Mengenverhältnis von Isocyanat der Formel R'-NCO zu Verbindung der Formel (III) kann breit variiert werden. Zweckmäßig sind 5 bis 100 Molprozent, vorzugsweise 10 bis 50 Molprozent Isocyanat der Formel R'-NCO, bezogen auf eingesetztes Sulfonamid der Formel (III).
In der Regel setzt die Reaktion anfangs verzögert ein. Überraschenderweise wurde gefunden, dass die Reaktionsgeschwindigkeit am Anfang wesentlich erhöht werden kann, wenn man als Vorlage eine Menge eines Sulfonylisocyanats, vorzugsweise eine katalytische Menge des Sulfonylisocyanats der Formel (IV), das auch hergestellt werden soll, einsetzt. Die zweckmäßige Menge kann in Vorversuchen bestimmt werden. In der Regel genügen 5 bis 20 Molprozent Verbindung, (IV) als Vorlage, bezogen auf die Menge an Sulfonamid (III), um einen verzögerten Einsatz der Reaktion weitgehend zu vermeiden.
Zur Phosgenierung kann man beispielsweise die Verbindung (III) in einem organischen Lösungsmittel zusammen mit dem Isocyanat der Formel R'-NCO vorlegen und Phosgen einleiten. Altenativ kann man das Isocyanat in einem organischen Lösungsmittel vorlegen und Phosgen einleiten sowie gleichzeitig kontinuierlich oder portionsweise Sulfonamid der Formel (III) zugeben. Beim letztgenannten Verfahren ist die Konzentration des Sulfonamids während der Reaktion verhältnismäßig gering, was den Anteil an Nebenprodukten reduziert. Bevorzugt ist die Verfahrensweise mit Vorlage des Isocyanats der Formel R'-NCO und eines Teils Sulfonylisocyanat (IV) in einem organischen Lösungsmittel und anschließendem Einleiten von Phosgen.

Die Reaktionstemperatur für die Phosgenierung ist beispielsweise im Bereich von 50 °C bis zum Siedepunkt des jeweiligen Lösungsmittels, vorzugsweise im Bereich von 100 °C bis zum Siedepunkt des jeweiligen Lösungsmittels, insbesondere 100 °C bis 180 °C, ganz besonders 120 bis 140 °C.

Die Reaktion kann unter Normaldruck, Überdruck oder Unterdruck durchgeführt werden. Zweckmäßig ist ein Phosgenpartialdruck im Bereich von 0,2 bis 20 bar, vorzugsweise 1 bis 6 bar.

Um einen möglichst guten Umsatz an Verbindung (III) zu erzielen, wird Phosgen vorzugsweise in einer Menge von 1 bis 4 Mol, besonders 1 bis 2,5 Mol pro Mol Verbindung der Formel (III) eingesetzt. Dabei wird während der Reaktion vorzugsweise stets ein Überschuß an Phosgen eingestellt. Der Gehalt an Phosgen in der Reaktionsmischung beträgt vorzugsweise 2 Gewichtsprozent, insbesondere mehr als 5 Gewichtsprozent in der Reaktionsmischung.

Zur Aufarbeitung des Reaktionsgemischs kann das Produkt (IV) nach üblichen Methoden isoliert werden oder aber vorzugsweise das Reaktionsgemisch nach Entfernen des Überschusses an Phosgen und des Isocyanats der Formel R'-NCO direkt für die Umsetzung zur Verbindung (I) (Kupplungsreaktion) eingesetzt werden. Ein Vorteil des erfindungsgemäßen Verfahrens besteht in der Möglichkeit einer technisch einfachen Aufarbeitung, d. h. mittels Destillation eines Teils des Lösungsmittels, gegebenenfalls unter reduziertem Druck, können gleichzeitig der Überschuß an Phosgen und das Isocyanat der Formel R'-NCO entfernt werden.

Mit der erfindungsgemäßen Phosgenierung (b1) werden in der Regel Ausbeuten von über 90% d. Th. an Verbindung (IV) erreicht, und das überraschenderweise auch für die Phosgenierung von Verbindungen (III) mit sterisch anspruchsvollen Resten (X*= Chlor, Brom, lod), die zu Nebenreaktionen neigen. Die Verbindungen (IV) können nach dem bevorzugten Verfahren hergestellt werden, ohne eine Aminbase als Katalysator einsetzen zu müssen. Die Reinheiten sind für eine Weiterverarbeitung ausreichend hoch. Eine Entfernung von Katalysator oder entsprechenden Salzen und deren Aufarbeitung ist entbehrlich. Die eingesetzten Lösungsmittel lassen sich technisch einfach abtrennen und für weitere Ansätze wiederverwenden. Das Verfahren ist wegen der genannten Merkmale ökonomisch und ökologisch vorteilhaft.

Die nach Stufe (b1) erhaltene Verbindung (IV) kann mit oder ohne Zwischenisolierung in einem organischen Lösungsmittel mit einem Amin der genannten Formel (V) zum Sulfonylharnstoff der Formel (I) oder dessen Salzen umgesetzt werden.

Standardmäßig können die Umsetzungen von Phenylsulfonylisocyanaten mit Aminopyrimidinen oder Aminotriazinen in einem organischen Lösungsmittel erfolgen. Als Lösungsmittel werden dabei in der Regel polare aprotische Lösungsmittel wie THF und Acetonitril empfohlen (siehe z. B. EP-A-0030138, Seiten 14-15). Die Umsetzungen mit Verbindungen der Formel (IV) und Aminen der Formel (V) sind jedoch hinsichtlich der erreichten Reaktionsgeschwindigkeiten, der Ausbeuten und der Reinheiten oftmals nicht befriedigend. Ausbeutesteigerungen und Verminderung der Reaktionszeiten können mittels Zusatz von Katalysatoren wie sterisch gehinderten Aminbasen erfolgen, die aber nach der Reaktion wieder abgetrennt werden müssen (siehe anfangs genannte Literaturstellen).

Erfindungsgemäß können genannte Nachteile vermieden werden, wenn man die Umsetzung in einem Lösungsmittelgemisch aus einem gegebenenfalls halogenierten aromatischen Kohlenwasserstoff mit einem Siedepunkt von mehr als 110°C, vorzugsweise 120-200 °C, insbesondere 130 bis 180 °C, und einem polaren aprotischen Lösungsmittel durchführt, wobei das Gewichtsverhältnis des unpolaren Lösungsmittel zum polaren Lösungsmittel vorzugsweise im Bereich von 20:1 bis 1:10, insbesondere von 10:1 bis 1:5, ganz besonders von 5:1 bis 1:1 ist.

Als vergleichsweise unpolare Lösungsmittel sind Toluol, Xylol, Chlorbenzol oder Dichlorbenzol oder deren Gemische, vorzugsweise o-, m- oder p-Xylol oder das übliche technische Xylol (technisches Gemisch der Xylole) geeignet.
Als polare aprotische Lösungsmittel sind vorzugsweise die zu Verfahrensschritt (a) genannten polaren Lösungsmittel geeignet, insbesondere die Essigsäurealkylester oder Acetonitril. Bevorzugt sind Gemische von Xylol/Essigsäureethylester oder Xylol/Acetonitril.

Die Reaktion wird bevorzugt bei einer Temperatur im Bereich von 0 bis 100°C, vorzugsweise 20 bis 80 °C, insbesondere 40 bis 70 °C durchgeführt.

Bezogen auf ein Mol Sulfonylisocyanat der Formel (IV) werden vorzugsweise 1 bis 1,2 Mol, insbesondere 1 bis 1,1 Mol, ganz besonders 1 bis 1,05 Mol Amin der Formel (V) eingesetzt.

Das erfindungsgemäße bevorzugte Verfahren nach Variante (c1) ermöglicht den Einsatz von äquivalenten Mengen oder nahezu äquivalenten Mengen an Verbindungen (IV) und (V). Bei anderer Wahl der Lösungsmittel, beispielsweise bei Verwendung eines Lösungsmittels anstelle des erfindungsgemäß zu verwendenden Lösungmittelgemischs wird in der Regel kein vollständiger Umsatz erreicht oder muß für einen Umsatz der Verbindung (IV) ein größerer Überschuss an Verbindung (V) eingesetzt werden. Außerdem ist mit nur einem Lösungsmittel die Reaktionsgeschwindigkeit oft unbefriedigend niedrig. Die erfindungsgemäße Verfahrensweise ergibt dagegen meist ein Produkt in hervorragenden Ausbeute und Reinheit.

Die Aufarbeitung des Reaktionsgemischs aus der Kupplung kann nach üblichen Methoden erfolgen, wobei die Sulfonylharnstoffe der Formel (I) entweder als Nichtsalze oder - nach Umsetzung mit Basen - als Salze isoliert werden können.

Besonders bevorzugt ist das Verfahren, bei dem die bevorzugten Verfahrensstufen (a1), (b1) und (c1) kombiniert durchgeführt werden. Bei Verwendung derselben Lösungsmittelgemische sind dabei auch Eintopfreaktionen möglich.

In den folgenden Beispielen beziehen sich Mengenangaben auf das Gewicht, sofern nicht speziell andere Definitionen angegeben sind.

### Beispiel 1a

### 2-Amidosulfonyl-4-iod-benzoesäuremethylester

Zu einer Lösung von 73 g 2-Chlorsulfonyl-4-iod-benzoesäuremethylester,(98,7%ig) in 189 g Xylol und 150 g Acetonitril werden bei 18-22 °C insgesamt 9,1 g Ammoniak innerhalb von 1 Stunde eingegast. Anschließend wird für 1 Stunde unter Rühren Stickstoff durch die Suspension geleitet. Restlicher Ammoniak wird durch Abdestillieren von wenig Lösungsmittel im Vakuum entfernt. Es wird auf Rückfluß erhitzt, heiß filtriert und der Rückstand erschöpfend mit frischem Acetonitril ausgekocht. Die vereinigten Filtrate werden destillativ eingeengt und der Rückstand kalt filtriert. Man erhält 66,8 g weißer Nadeln von 2-Amidosulfonyl-4-iod-benzoesäuremethylester.

### Beispiel 1b (Vergleichsbeispiel)

Führt man die Reaktion gemäß Beispiel 1a in reinem Xylol anstelle des Xylol/Acetonitril-Gemischs durch, erhält man nach einer Reaktionszeit von 6 Stunden und ansonsten identischen Bedingungen eine Ausbeute von 56,6 g 2-Amidosulfonyl-4-iod-benzoesäuremethylester bei einer Reinheit von nur 83,3% (Ausbeute 69,1% d. Th.).

### Beispiel 2a

### 2-Amidosulfonyl-4-iod-benzoesäuremethylester

Eine Lösung von 144,2 g 2-Chlorsulfonyl-4-iod-benzoesäuremethylester in 217,4 g Xylol und 150 g Ethylacetat wird unter Rühren innerhalb von 4 Stunden bei 35 bis 37 °C zu einer gesättigten Lösung von Ammoniak in Essigsäureethylester (156,5 g) dosiert. Zeitgleich werden insgesamt 16,7 g Ammoniak mit einer Geschwindigkeit eingeleitet, wie Ammoniak verbraucht wird. Es wird 1 Stunde nachgerührt, überschüssiges Ammoniak durch Andestillieren im Vakuum entfernt, auf Rückfluß erhitzt und heiß filtriert. Der Filterkuchen wird mit siedendem Ethylacetat erschöpfend ausgekocht. Dann wird Ethylacetat weitgehend abdestilliert und die verbleibende xylolische Suspension des Produktes auf 20 °C abgekühlt. Nach Filtration und Trocknen im Vakuum erhält man 132,2 g 2-Amidosulfonyl-4-iod-benzoesäuremethylester (Reinheit 99,3 %)

### Beispiel 2b (Vergleichsbeispiel)

Führt man die Reaktion nach Beispiel 2a in einem Gemisch aus Xylol/Methanol (4:1) durch, so ergibt sich unter ansonsten gleichen Bedingungen nach der Aufarbeitung eine Ausbeute von 118,8 g 2-Amidosulfonyl-4-iod-benzoesäuremethylester bei einer Reinheit von 71% (Ausbeute 62% d. Th.)

### Beispiel 3

### 2-Amidosulfonyl-4-iod-benzoesäuremethylester

Es wird wie in Beispiel 2a verfahren, wobei jedoch anstelle einer Lösung des Sulfochlorids in 217,4 g Xylol und 150 g Essigsäureethylester eine Lösung des Sulfochlorids in 300 g Xylol und 75 g Essigsäureethylester eingesetzt wird. Dabei werden 130 g 2-Amidosulfonyl-4-iod-benzoesäuremethylester (Reinheit 99 %) erhalten.

### Beispiel 4

### 2-Amidosulfonyl-4-iod-benzoesäuremethylester

Es wird wie in Beispiel 2a verfahren, wobei jedoch anstelle einer Lösung des Sulfochlorids in 217,4 g Xylol und 150 g Essigsäureethylester eine Lösung des Sulfochlorids in 300 g Xylol und 75 g Acetonitril eingesetzt wird. Dabei werden 131 g 2-Amidosulfonyl-4-iod-benzoesäuremethylester (Reinheit 99 %) erhalten.

### Beispiel 5a

### 4-lod-2-isocyanatosulfonyl-benzoesäuremethylester

150 ml trockenes Xylol und 6,84 ml n-Butylisocyanat werden unter Stickstoff-Schutzgas auf 126-128 °C erhitzt. Danach wird zunächst Phosgen (insgesamt 50 g) über die Oberfläche zugeführt. Der Phosgenstrom wird so geregelt, das die Temperatur des Reaktionsgemisches bei 126-128 °C bleibt. Gegebenenfalls wird die Phosgendosierung reduziert oder unterbrochen. Aus dem Reaktionsgemisch entweichendes Phosgen wird mittels eines auf -20 °C gekühlten Kühlers kondensiert und ins Reaktionsgemisch zurückgeleitet.
Während der Einleitung des Phosgens werden 90 g 2-Amidosulfonyl-4-iod-benzoesäuremethylester als Suspension in 336 ml Xylol portionsweise innerhalb von 20 Stunden unter Rühren zugegeben. Nach weiteren 10 Stunden Rühren unter einer mit Phosgen gesättigten Atmosphäre werden bei gleicher Reaktionstemperatur 300 ml eines Gemisches von n-Butylisocyanat und Xylol unter reduziertem Druck (100 bis 120 mbar, 78 bis 80 °C) abdestilliert, wobei während der Destillation wieder 400 ml trockenes Xylol in drei Portionen zugegeben werden. Die Ausbeute der Titelverbindung beträgt 92,8 % d.Th.

### Beispiel 5b (Vergleichsbeispiel)

Das Verfahren gemäß Beispiel 5a wird ohne Zusatz eines Alkylisocyanates (z. B. Butylisocyanat) wiederholt, jedoch findet dabei keine Umsetzung des zugefügten 2-Amidosulfonyl-4-iod-benzoesäuremethylesters statt.

### Beispiel 6a

### 4-lod 2-isocyanatosulfonyl-benzoesäuremethylester (bei Verwendung einer Vorlage)

Zu einer Lösung von 4-lod-2-isocyanatosulfonyl-benzoesäuremethylester (19,7 g als 14,8 %igen Lösung in Xylol) werden unter Stickstoff-Schutzgas 3,73 ml n-Butylisocyanat und 250 ml Xylol gegeben. Die Mischung wird unter Rühren auf 126-128 °C erhitzt, und Phosgen wird über die Oberfläche zugegeben. Der Phosgenstrom wird so geregelt, das die Temperatur des Reaktionsgemisches bei 126 - 128 °C bleibt. Gegebenenfalls wird die Phosgendosierung reduziert oder unterbrochen. Aus dem Reaktionsgemisch entweichendes Phosgen wird mittels eines auf -20 °C gekühlten Kühlers kondensiert und ins Reaktionsgemisch zurückgeleitet. Gleichzeitig werden 90 g 2-Amidosulfonyl-4-iod-benzoesäuremethylester; als Suspension in 216 ml Xylol portionsweise innerhalb von 10 Stunden unter Rühren zugegeben. Nach weiteren 10 Stunden unter Rühren in einer mit Phosgen gesättigten Atmosphäre bei gleicher Reaktionstemperatur werden 300 ml eines Gemisches von n-Butylisocyanat und Xylol unter reduziertem Druck abdestilliert, wobei während der Destillation 433 ml trockenes Xylol wieder zugeführt werden. Die Ausbeute der Titelverbindung beträgt 94,8 % d.Th.

### Beispiel 6b (Vergleichsbeispiel)

Das Verfahren gemäß Beispiel 6a wird entsprechend ohne Vorlage einer Lösung des 4-lod-2-isocyanatosulfonyl-benzoesäuremethylesters wiederholt. Nach einer Reaktionszeit von 20 Stunden ist die Reaktion noch unvollständig, und die Ausbeute an 4-lod 2-isocyanatosulfonyl-benzoesäuremethylester beträgt 86% d. Th.

### Beispiel 7a (Kupplung)

### 4-Iod-2-{N-[N-(4-methoxy-6-methyl-1,3,5-triazin-2-yl)-aminocarbonyl]-aminosulfonyl}-benzoesäuremethylester

Zur Suspension von 84,5 g 2-Amino-4-methoxy-6-methyl-1,3,5-triazin in 670 g Ethylacetat wird unter Schutzgas innerhalb von 4 Stunden eine 14,5 %ige Lösung (1465 g) von 4-lod-2-isocyanatosulfonyl-benzoesäuremethylester in Xylol mit einer konstanten Rate bei 50 °C zugegeben. Nach beendeter Zugabe wird ca. 4 Stunden bei derselben Temperatur gerührt, bevor das Ethylacetat im Vakuum (80 - 60 mbar, T = 50 °C) abdestilliert wird. Die verbleibende Suspension wird abgesaugt, der Feststoff mit verdünnter Salzsäure mehrfach gewaschen und getrocknet; gegebenenfalls kann der Salzsäure Aceton beigesetzt werden. Es werden 297 g (Gehalt > 98 %) der Titelverbindung erhalten; Ausbeute 99,2 % d. Th.

### Beispiel 7b (Vergleichsbeispiel)

Das Verfahren gemäß Beispiel 7a wird entsprechend mit reinem Xylol als Lösungsmittel wiederholt. Nach 24 Stunden ist die Reaktion noch unvollständig. Die Titelverbindung wird in einer Ausbeute von 81 % d. Th. und einer Reinheit von 89% erhalten.

## Patentansprüche

1. Verfahren zur Herstellung der Verbindung der Formel (I) oder deren Salze, 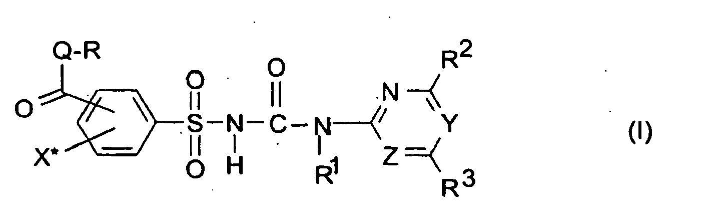 worin
Q Sauerstoff, Schwefel oder -N(R⁴)-,
X* Halogen,
Y,Z unabhängig voneinander CH oder N, wobei Y und Z nicht gleichzeitig CH sind,
R Wasserstoff, (C₁-C₁₂)-Alkyl; (C₂-C₁₀)-Alkenyl; (C₂-C₁₀)-Alkinyl; (C₁-C₆)-Alkyl, das ein- bis vierfach durch Reste aus der Gruppe Halogen, (C₁-C₄)-Alkoxy, (C₁-C₄)-Alkylthio, CN, [(C₁-C₄)-Alkoxy]-carbonyl und (C₂-C₆)-Alkenyl substituiert ist; oder (C₃-C₈)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist; (C₅-C₈)-Cycloalkenyl; Phenyl-(C₁-C₄)-alkyl, das im Phenylrest unsubstituiert oder durch einen oder mehrere Reste aus der Gruppe Halogen, (C₁-C₄)-Alkyl, (C₁-C₄)-Alkoxy, (C₁-C₄)-Haloalkyl, (C₁-C₄)-Alkylthio, [(C₁-C₄)-Alkoxy]-carbonyl, [(C₁-C₄)-Alkyl]-carbonyloxy, Carbonamid, [(C₁-C₄)-Alkyl]-carbonylamino, [(C₁-C₄)-Alkyl]-aminocarbonyl, Di-[(C₁-C₄)-alkyl]-aminocarbonyl und Nitro substituiert ist; oder einen Rest der Formeln A-1 bis A-10 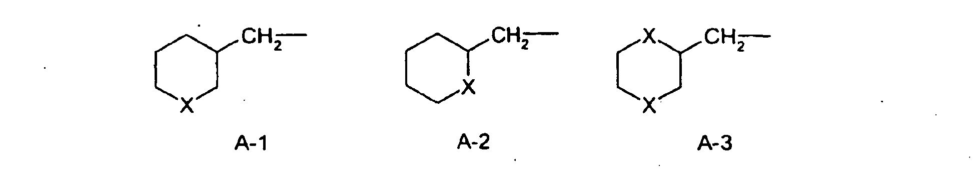 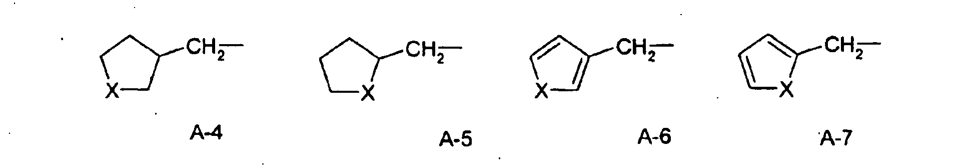 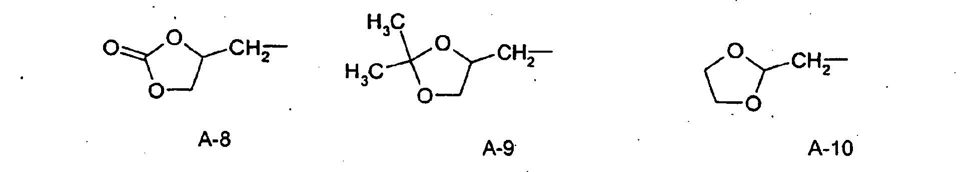
worin
X O, S, S(O) oder SO₂;
R¹ Wasserstoff oder (C₁-C₃)-Alkyl;
R² Wasserstoff, Halogen, (C₁-C₃)-Alkyl oder (C₁-C₃)-Alkoxy, wobei jeder beiden letztgenannten Reste unsubstituiert oder ein- oder mehrfach durch Halogen oder (C₁-C₃)-Alkoxy substituiert ist;
R³ Wasserstoff, Halogen, (C₁-C₃)-Alkyl, (C₁-C₃)-Alkoxy oder (C₁-C₃)-Alkylthio,
wobei jeder der letztgenannten drei Reste unsubstituiert oder ein- oder mehrfach durch Halogen oder ein- oder zweifach durch (C₁-C₃)-Alkoxy oder (C₁-C₃)-Alkylthio substituiert ist; oder einen Rest der Formel NR⁵R⁶, (C₃-C₆)-Cycloalkyl, (C₂-C₄)-Alkenyl, (C₂-C₄)-Alkinyl, (C₃-C₄)-Alkenyloxy oder (C₃-C₄)-Alkinyloxy;
R⁴ Wasserstoff, (C₁-C₄)-Alkyl oder (C₁-C₄)-Alkoxy und
R⁵ und R⁶ unabhängig voneinander Wasserstoff, (C₁-C₄)-Alkyl, (C₃-C₄)-Alkenyl, (C₁-C₄)-Haloalkyl oder (C₁-C₄)-Alkoxy bedeuten,
**dadurch gekennzeichnet, daß** man
a) eine Verbindung der Formel (II) 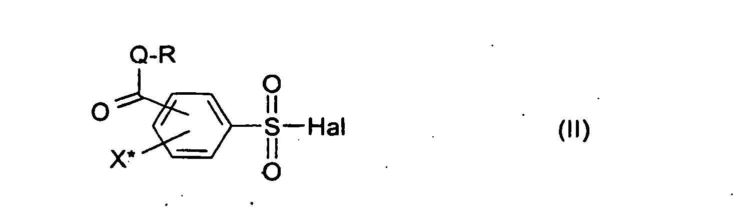 worin Hal ein Halogenatom bedeutet und R, Q und X* wie in Formel (I) definiert sind,
durch Ammonolyse mit Ammoniak zur Verbindung der Formel (III) umsetzt, 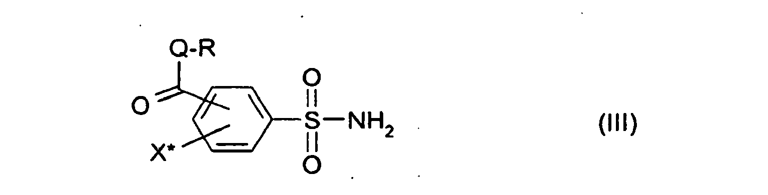 worin R, Q und X* wie in Formel (I) definiert sind,
oder vorzugsweise
(a1) die Verbindung der Formel (II) zur Verbindung der Formel (III) ammonolysiert und dabei die Umsetzung in einem organischen Lösungsmittelgemisch enthaltend
(1) einen oder mehrere gegebenenfalls halogenierte aromatische Kohlenwasserstoffe [Lösungsmittel (1)] und
(2) ein oder mehrere polare aprotische Lösungsmittel [Lösungsmittel (2)], im Gewichtsverhältnis Lösungsmittel (1):Lösungsmittel (2) von 20:1 bis 1:1, vorzugsweise von 10:1 bis 1,4:1 durchführt,
(b1) die erhaltene Verbindung (III) mit oder ohne Zwischenisolierung mit Phosgen zum Phenylsulfonylisocyanat der Formel (IV) umsetzt, 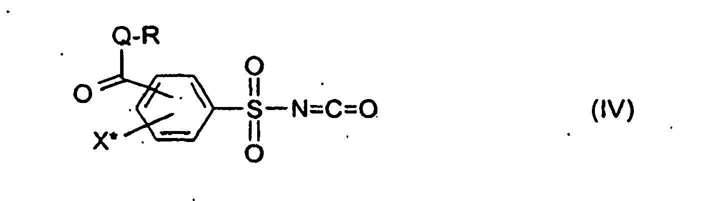 worin R, Q und X* wie in Formel (I) definiert sind,
und dabei die Umsetzung mit Phosgen in einem organischen Lösungsmittel in Gegenwart eines Isocyanats der Formel R'-NCO als Katalysator, wobei R' ein Kohlenwasserstoffrest ist, der unsubstituiert oder substituiert ist, oder eines Gemisches mehrerer dieser Isocyanate als Katalysator ohne Zugabe einer Aminbase oder anderen Base als Ko-katalysator durchführt,
und
(c) die erhaltene Verbindung (IV) mit oder ohne Zwischenisolierung in einem organischen Lösungsmittel mit einem Amin der Formel (V) 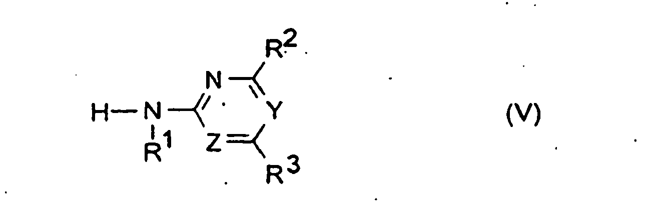 worin R¹, R², R³, Y und Z wie in Formel (I) definiert sind,
zum Sulfonylharnstoff der Formel (I) oder dessen Salzen umsetzt,
oder vorzugsweise
(c1) die erhaltene Verbindung (IV) mit oder ohne Zwischenisolierung in einem organischen Lösungsmittel mit einem Amin der Formel (V) zur Verbindung der Formel (I) oder deren Salzen umsetzt und dabei die Umsetzung in einem Lösungsmittelgemisch aus einem gegebenenfalls halogenierten aromatischen Kohlenwasserstoff mit einem Siedepunkt von mehr als 110 °C und einem polaren aprotischen Lösungsmittel durchführt.

2. Verfahren nach Anspruch 1, **dadurch gekennzeichnet, daß** in Formel (I) oder deren Salzen
Q ein Sauerstoffatom,
X* ein Halogenatom,
R (C₁-C₄)-Alkyl; (C₂-C₄)-Alkenyl; (C₂-C₄)-Alkinyl; (C₁-C₄)-Haloalkyl oder (C₁-C₄)-Alkoxy(C₁-C₄)-alkyl,
R¹ ein Wasserstoffatom,
R² (C₁-C₄)-Alkyl oder (C₁-C₄)-Alkoxy,
R³ (C₁-C₄)-Alkyl oder (C₁-C₄)-Alkoxy,
Y ein Stickstoffatom oder eine Gruppe der Formel CH und
Z ein Stickstoffatom
bedeuten.

3. Verfahren nach Anspruch 2, **dadurch gekennzeichnet, daß** in Formel (I) oder deren Salzen
X* ein Iodatom,
R Methyl oder Ethyl,
R² Methoxy,
R³ Methyl,
Y ein Stickstoffatom und
Z ein Stickstoffatom
bedeuten.

4. Verfahren nach einem der Ansprüche 1 bis 3, **dadurch gekennzeichnet, daß** die Umsetzung in der Stufe (a) gemäß Variante (a1) in einem organischen Lösungsmittelgemisch, enthaltend
(1) einen oder mehrere gegebenenfalls halogenierte aromatische Kohlenwasserstoffe aus der Gruppe Xylol, Toluol, Chlorbenzol und Dichlorbenzol, [Lösungsmittel (1)] und
(2) ein oder mehrere polare aprotische Lösungsmittel [Lösungsmittel (2)] aus der Gruppe enthaltend Nitrile, Ester, Ether, Amide, Ketone und Gemische aus zwei oder mehreren der polaren Lösungsmitteln,
durchgeführt wird.

5. Verfahren nach einem der Ansprüche 1 bis 4, **dadurch gekennzeichnet, daß** die Umsetzung in der Stufe (b1) in Gegenwart eines oder mehrerer Isocyanate aus der Gruppe N-Alkylisocyanate, N-Cycloalkylisocyanate und N-Arylisocyanate als Katalysator durchgeführt wird.

6. Verfahren nach einem der Ansprüche 1 bis 5, **dadurch gekennzeichnet, daß** die Umsetzung in der Stufe (b1) in Gegenwart von n-Butylisocyanat oder Cyclohexylisocyanat als Katalysator durchgeführt wird.

7. Verfahren nach einem der Ansprüche 1 bis 6, **dadurch gekennzeichnet, daß** die Umsetzung in der Stufe (c1) in Xylol/Essigsäureethylester oder Xylol/Acetonitril durchgeführt wird.

8. Verfahren zur Herstellung von Verbindungen der Formel (IV), 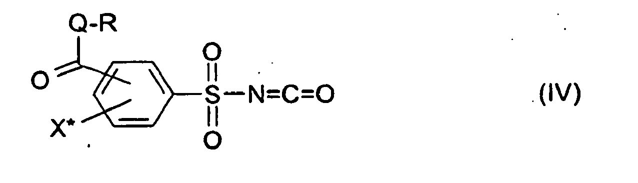 worin R und Q wie in Formel (I) nach einem der Ansprüche 1 bis 3 definiert sind,
**dadurch gekennzeichnet, dass** man eine Verbindung der Formel (III), 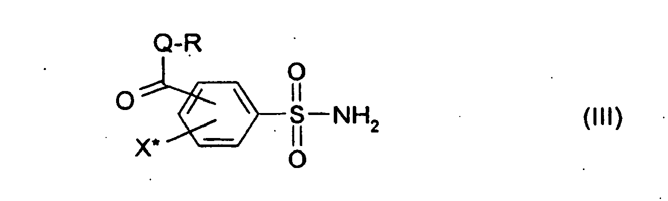 worin R, Q und X* wie in Formel (IV) definiert sind,
mit Phosgen in einem organischen Lösungsmittel in Gegenwart eines oder mehrerer Isocyanate der Formel R'-NCO als Katalysator, wobei R' ein Kohlenwassefstoffrest ist, der unsubstituiert oder substituiert ist, ohne Zugabe einer Aminbase oder anderen Base als Ko-katalysator zum Phenylsulfonylisocyanat der Formel (IV) umsetzt.

9. Verfahren nach Anspruch 8, **dadurch gekennzeichnet, dass** man die zur Herstellung der Verbindung der Formel (IV) zu verwendende Verbindung der Formel (III), 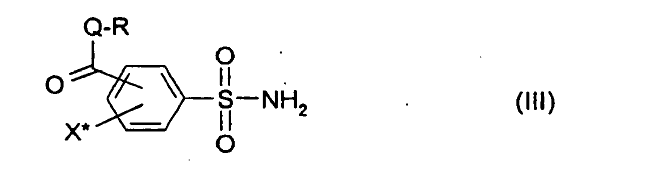 worin R, Q und X* wie in Formel (IV) definiert sind,
herstellt, indem man eine Verbindung der Formel (II), 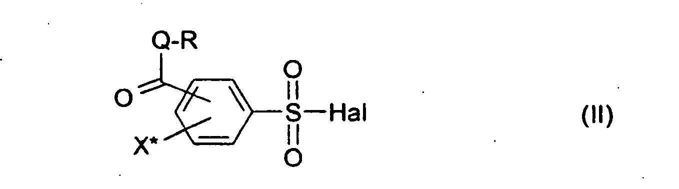 worin R, Q und X* wie in Formel (III) definiert sind,
durch Ammonolyse mit Ammoniak zur Verbindung der Formel (III) umsetzt.

10. Verfahren nach Anspruch 9, **dadurch gekennzeichnet, dass** die Umsetzung der Verbindung der Formel (II) durch Ammonolyse in einem organischen Lösungsmittelgemisch, enthaltend
(1) einen oder mehrere gegebenenfalls halogenierte aromatische Kohlenwasserstoffe und
(2) ein oder mehrere polare aprotische Lösungsmittel, im Gewichtsverhältnis Lösungsmittel (1):Lösungsmittel (2) von 20:1 bis 1:1, vorzugsweise von 10:1 bis 1,4:1
durchgeführt wird,

11. Verfahren nach Anspruch 10, **dadurch gekennzeichnet, dass** die Umsetzung der Verbindung der Formel (II) durch ammonolyse in einem organischen Lösungsmittelgemisch, enthaltend
(1) einen oder mehrere gegebenenfalls halogenierte aromatische Kohlenwasserstoffe aus der Gruppe Xylol, Toluol, Chlorbenzol und Dichlorbenzol, und
(2) ein oder mehrere polare aprotische Lösungsmittel aus der Gruppe enthaltend Nitrile, Ester, Ether, Amide, Ketone und Gemische aus zwei oder mehreren der polaren Lösungsmitteln,
durchgeführt wird.

12. Verfahren zur Herstellung von Verbindungen der Formel (I) oder deren Salzen, wobei die Verbindungen gemäß einem der Ansprüche 1 bis 3 definiert sind, **dadurch gekennzeichnet, daß** man
(b1) eine Verbindung der Formel (III), 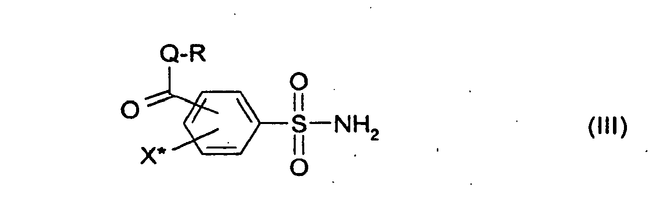 worin R, Q und X* wie in Formel (I) definiert sind,
mit Phosgen zum Phenylsulfonylisocyanat der Formel (IV) umsetzt, 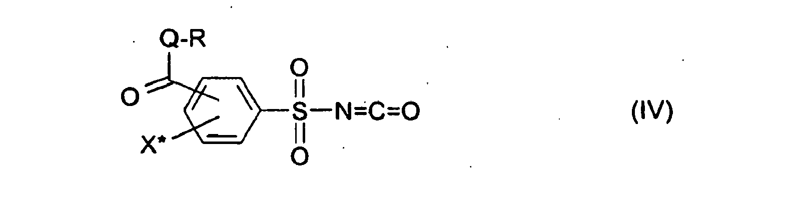 worin R, Q und X* wie in Formel (I) definiert sind,
und dabei die Umsetzung mit Phosgen in einem organischen Lösungsmittel in Gegenwart eines Isocyanats der Formel R'-NCO als Katalysator, wobei R' ein Kohlenwasserstoffrest ist, der unsubstituiert oder substituiert ist, oder eines Gemisches mehrerer dieser Isocyanate als Katalysator ohne Zugabe einer Aminbase oder einer anderen Base als Kakatalysator durchführt; und
(c) die erhaltene Verbindung (IV) mit oder ohne Zwischenisolierung in einem organischen Lösungsmittel mit einem Amin der Formel (V) 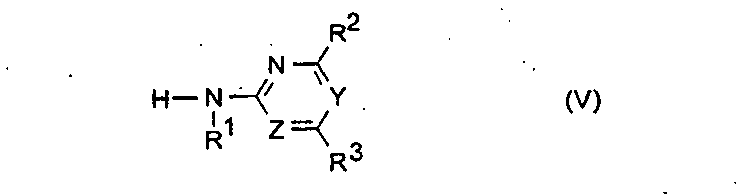 worin R¹, R², R³, Y und Z wie in Formel (I) definiert sind,
zum Sulfonylharnstoff der Formel (I) oder dessen Salzen umsetzt, oder vorzugsweise
(c1) die erhaltene Verbindung (IV) mit oder ohne Zwischenisolierung in einem organischen Lösungsmittel mit einem Amin der Formel (V) zur Verbindung der Formel (I) oder deren Salzen umsetzt und dabei die Umsetzung in einem Lösungsmittelgemisch aus einem gegebenenfalls halogenierten aromatischen Kohlenwasserstoff mit einem Siedepunkt von mehr als 110 °C und einem polaren aprotischen Lösungsmittel durchführt.

13. Verfahren nach Anspruch 12, **dadurch gekennzeichnet, daß** die Umsetzung in der Stufe (b1) in Gegenwart eines oder mehrerer Isocyanate aus der Gruppe N-Alkylisocyanate, N-Cycloalkylisocyanate und N-Arylisocyanate als Katalysator durchgeführt wird.

14. Verfahren nach Anspruch 12 oder 13, **dadurch gekennzeichnet, daß** die Umsetzung in Stufe (c1) in Xylol/Essigsäureethylester oder Xylol/Acetonitril durchgeführt wird.

15. Verfahren nach einem der Ansprüche 1 bis 14, **dadurch gekennzeichnet, dass**
am Phenylring
- die Gruppe -CO-Q-R in Orthostellung zur substituierten Sulfonylgruppe steht und
- die Gruppe X* in Parastellung zur Gruppe -CO-Q-R steht.

16. Verfahren nach einem der Ansprüche 1 bis 15, **dadurch gekennzeichnet, dass**
der Rest X* am Phenylring Iod bedeutet.

## Claims

1. A process for preparing the compound of the formula (I) or salts thereof 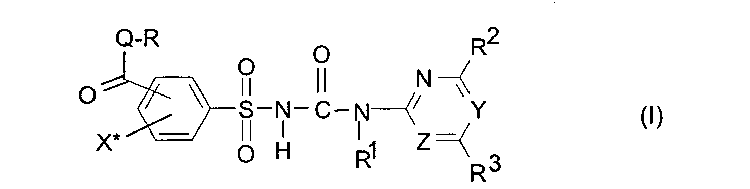 in which
Q is oxygen, sulfur or -N(R⁴)-,
X* is halogen,
Y,Z independently of one another are CH or N, where Y and Z are not simultaneously CH,
R is hydrogen, (C₁-C₁₂)-alkyl; (C₂-C₁₀)-alkenyl; (C₂-C₁₀)-alkynyl; (C₁-C₆)-alkyl, which is mono- to tetrasubstituted by radicals selected from the group consisting of halogen, (C₁-C₄)-alkoxy, (C₁-C₄)-alkylthio, CN, [(C₁-C₄)-alkoxy]carbonyl and (C₂-C₆)-alkenyl; or (C₃-C₈)-cycloalkyl, which is unsubstituted or substituted by radicals selected from the group consisting of (C₁-C₄)-alkyl, (C₁-C₄)-alkoxy, (C₁-C₄)-alkylthio and halogen; (C₅-C₈)-cycloalkenyl; phenyl-(C₁-C₄)-alkyl, which is unsubstituted in the phenyl radical or substituted in the phenyl radical by one or more radicals selected from the group consisting of halogen, (C₁-C₄)-alkyl, (C₁-C₄)-alkoxy, (C₁-C₄)-haloalkyl, (C₁-C₄)-alkylthio, [(C₁-C₄)-alkoxy]carbonyl, [(C₁-C₄)-alkyl]carbonyloxy, carboxamide, [(C₁-C₄)-alkyl]carbonyl-amino, [(C₁-C₄)-alkyl]aminocarbonyl, di[(C₁-C₄)-alkyl]aminocarbonyl and nitro; or is a radical of the formulae A-1 to A-10 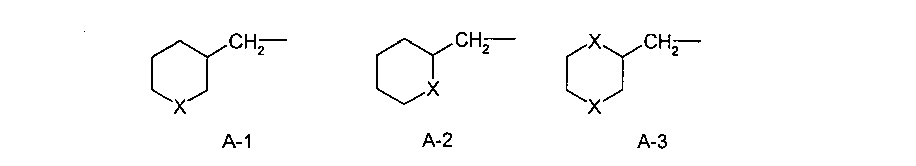 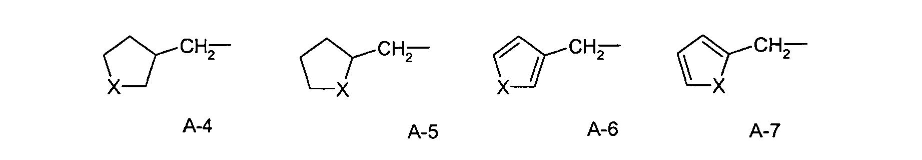 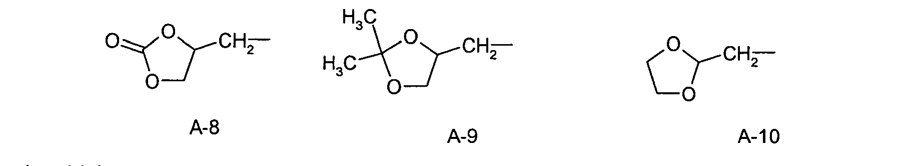 in which
X is O, S, S(O) or SO₂;
R¹ is hydrogen or (C₁-C₃)-alkyl;
R² is hydrogen, halogen, (C₁-C₃)-alkyl or (C₁-C₃)-alkoxy, where each of the two lastmentioned radicals is unsubstituted or mono- or polysubstituted by halogen or (C₁-C₃)-alkoxy;
R³ is hydrogen, halogen, (C₁-C₃)-alkyl, (C₁-C₃)-alkoxy or (C₁-C₃)-alkylthio, where each of the three lastmentioned radicals is unsubstituted or mono- or polysubstituted by halogen or mono- or disubstituted by (C₁-C₃)-alkoxy or (C₁-C₃)-alkylthio; or is a radical of the formula NR⁵R⁶, (C₃-C₆)-cycloalkyl, (C₂-C₄)-alkenyl, (C₂-C₄)-alkynyl, (C₃-C₄)-alkenyloxy or (C₃-C₄)-alkynyloxy;
R⁴ is hydrogen, (C₁-C₄)-alkyl or (C₁-C₄)-alkoxy and
R⁵ and R⁶ independently of one another are hydrogen, (C₁-C₄)-alkyl, (C₃-C₄)-alkenyl, (C₁-C₄)-haloalkyl or (C₁-C₄)-alkoxy,
which comprises
a) converting a compound of the formula (II) 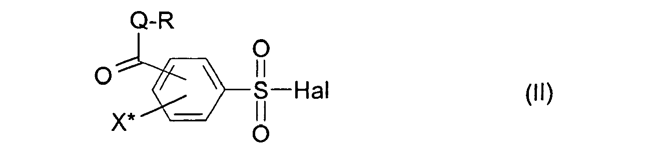 in which Hal is a halogen atom and R, Q and X* are as defined in formula (I),
by ammonolysis with ammonia into the compound of the formula (III) 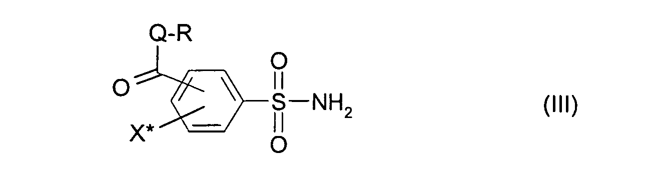 in which R, Q and X* are as defined in formula (I),
or preferably
(a1) ammonolyzing the compound of the formula (II) to give the compound of the formula (III) and carrying out the reaction in an organic solvent mixture comprising
(1) one or more optionally halogenated aromatic hydrocarbons [solvent (1)] and
(2) one or more polar aprotic solvents [solvent (2)]
in a weight ratio of solvent (1):solvent (2) of 20:1 to 1:1, preferably of 10:1 to 1.4:1,
(b1) reacting the resulting compound (III) with or without intermediate isolation with phosgene to give the phenylsulfonyl isocyanate of the formula (IV) 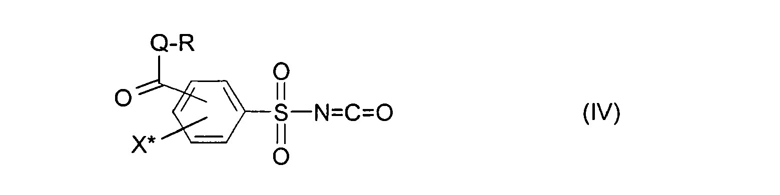 in which R, Q and X* are as defined in formula (I),
and carrying out the reaction with phosgene in an organic solvent in the presence of an isocyanate of the formula R'-NCO as catalyst, where R' is a hydrocarbon radical which is unsubstituted or substituted, or a mixture of a plurality of these isocyanates as catalyst without addition of an amine base or another base as cocatalyst,
and
(c) reacting the resulting compound (IV) with or without intermediate isolation in an organic solvent with an amine of the formula (V) 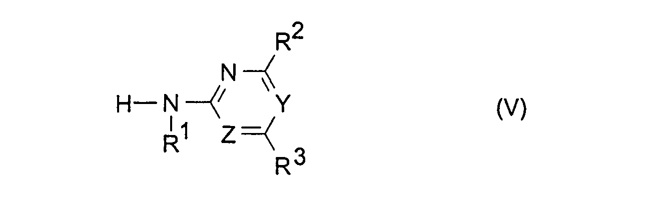 in which R¹, R², R³, Y and Z are as defined in formula (I)
to give the sulfonylurea of the formula (I) or a salt thereof, or preferably
(c1) reacting the resulting compound (IV) with or without intermediate isolation in an organic solvent with an amine of the formula (V) to give the compound of the formula (I) or a salt thereof, and carrying out the reaction in a solvent mixture of an optionally halogenated aromatic hydrocarbon having a boiling point of more than 110°C and a polar aprotic solvent.

2. The process as claimed in claim 1, wherein in the formula (I) or a salt thereof
Q is an oxygen atom,
X* is a halogen atom,
R is (C₁-C₄)-alkyl; (C₂-C₄)-alkenyl; (C₂-C₄)-alkynyl; (C₁-C₄)-haloalkyl or (C₁-C₄)-alkoxy(C₁-C₄)-alkyl,
R¹ is a hydrogen atom,
R² is (C₁-C₄)-alkyl or (C₁-C₄)-alkoxy,
R³ is (C₁-C₄)-alkyl or (C₁-C₄)-alkoxy,
Y is a nitrogen atom or a group of the formula CH and
Z is a nitrogen atom.

3. The process as claimed in claim 2, wherein in the formula (I) or a salt thereof
X* is an iodine atom,
R is methyl or ethyl,
R² is methoxy,
R³ is methyl,
Y is a nitrogen atom and
Z is a nitrogen atom.

4. The process as claimed in any of claims 1 to 3, wherein the reaction in step (a) according to variant (a1) is carried out in an organic solvent mixture comprising
(1) one or more optionally halogenated aromatic hydrocarbons selected from the group consisting of xylene, toluene, chlorobenzene and dichlorobenzene [solvent (1)] and
(2) one or more polar aprotic solvents [solvent (2)] selected from the group consisting of nitriles, esters, ethers, amides, ketones and mixtures of two or more of the polar solvents.

5. The process as claimed in any of claims 1 to 4, wherein the reaction in step (b1) is carried out in the presence of one or more isocyanates selected from the group consisting of N-alkyl isocyanates, N-cycloalkyl isocyanates and N-aryl isocyanates as catalyst.

6. The process as claimed in any of claims 1 to 5, wherein the reaction in step (b1) is carried out in the presence of n-butyl isocyanate or cyclohexyl isocyanate as catalyst.

7. The process as claimed in any of claims 1 to 6, wherein the reaction in step (c1) is carried out in xylene/ethyl acetate or xylene/acetonitrile.

8. A process for preparing compounds of the formula (IV) 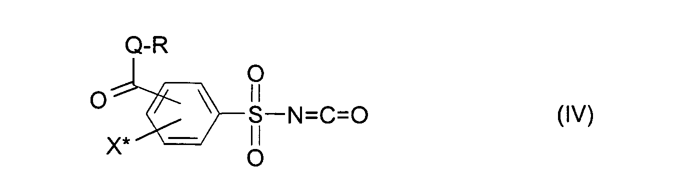 in which R and Q are as defined in formula (I) as set forth in any of claims 1 to 3,
which comprises reacting a compound of the formula (III) 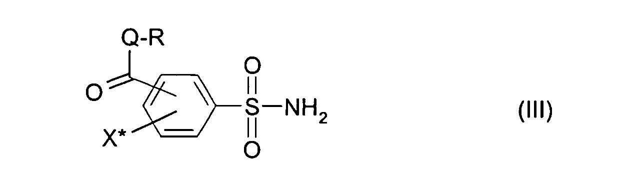 in which R, Q and X* are as defined in formula (IV)
with phosgene in an organic solvent in the presence of one or more isocyanates of the formula R'-NCO as catalyst, where R' is an unsubstituted or substituted hydrocarbon radical, without addition of an amine base or any other base as cocatalyst, to give the phenylsulfonyl isocyanate of the formula (IV).

9. The process as claimed in claim 8, wherein the compound, to be used for preparing the compound of the formula (IV), of the formula (III) 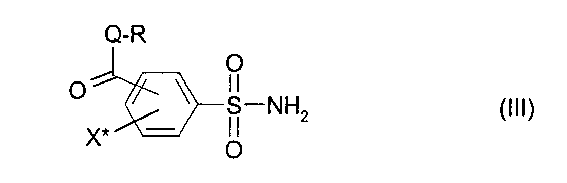 in which R, Q and X* are as defined in formula (IV)
is prepared by reacting a compound of the formula (II) 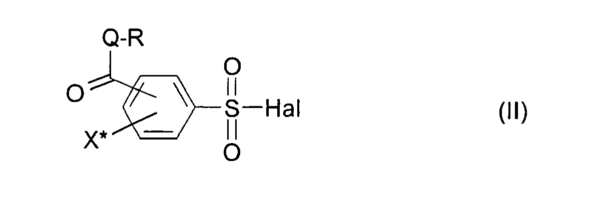 in which R, Q and X* are as defined in formula (III)
by ammonolysis with ammonia to give the compound of the formula (III).

10. The process as claimed in claim 9, wherein the reaction of the compound of the formula (II) is carried out by ammonolysis in an organic solvent mixture comprising
(1) one or more optionally halogenated aromatic hydrocarbons and
(2) one or more polar aprotic solvents,
in a weight ratio of solvent (1):solvent (2) of from 20:1 to 1:1, preferably from 10:1 to 1.4:1.

11. The process as claimed in claim 10, wherein the reaction of the compound of the formula (II) is carried out by ammonolysis in an organic solvent mixture comprising
(1) one or more optionally halogenated aromatic hydrocarbons from the group consisting of xylene, toluene, chlorobenzene and dichlorobenzene, and
(2) one or more polar aprotic solvents from the group consisting of nitriles, esters, ethers, amides, ketones and mixtures of two or more of the polar solvents.

12. A process for preparing compounds of the formula (I) or salts thereof, wherein the compounds are defined as set forth in any of claims 1 to 3, which comprises
(b1) reacting a compound of the formula (III) 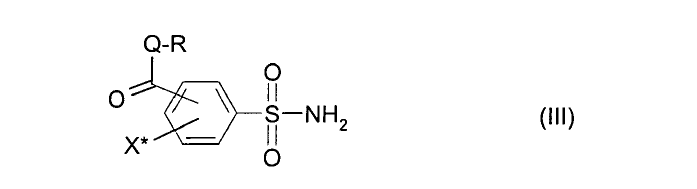 in which R, Q and X* are as defined in formula (I)
with phosgene to give the phenylsulfonyl isocyanate of the formula (IV) 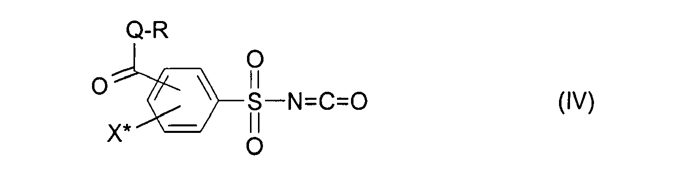 in which R, Q and X* are as defined in formula (I),
and carrying out the reaction with phosgene in an organic solvent in the presence of an isocyanate of the formula R'-NCO as catalyst,
where R' is a hydrocarbon radical which is unsubstituted or substituted, or a mixture of a plurality of these isocyanates as catalyst without addition of an amine base or another base as cocatalyst,
and
(c) reacting the resulting compound (IV) with or without intermediate isolation in an organic solvent with an amine of the formula (V) 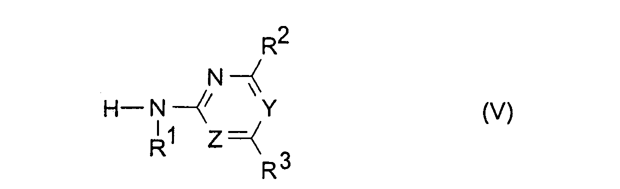 in which R¹, R², R³, Y and Z are as defined in formula (I)
to give the sulfonylurea of the formula (I) or a salt thereof, or preferably
(c1) reacting the resulting compound (IV) with or without intermediate isolation in an organic solvent with an amine of the formula (V) to give the compound of the formula (I) or a salt thereof, and carrying out the reaction in a solvent mixture of an optionally halogenated aromatic hydrocarbon having a boiling point of more than 110°C and a polar aprotic solvent.

13. The process as claimed in claim 12, wherein the reaction in step (b1) is carried out in the presence of one or more isocyanates from the group consisting of N-alkyl isocyanates, N-cycloalkyl isocyanates and N-aryl isocyanates as catalyst.

14. The process as claimed in claim 12 or 13, wherein the reaction in step (c1) is carried out in xylene/ethyl acetate or xylene/acetonitrile.

15. The process as claimed in any of claims 1 to 14, wherein on the phenyl ring
- the group -CO-Q-R is ortho to the substituted sulfonyl group and
- the group X* is para to the group -CO-Q-R.

16. The process as claimed in any of claims 1 to 15, wherein the radical X* at the phenyl ring is iodine.

## Revendications

1. Procédé pour la préparation du composé de la formule (I) ou de ses sels, 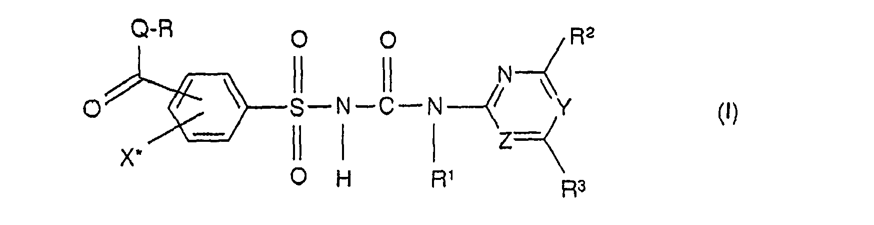 dans laquelle
Q représente un atome d'oxygène, de soufre ou -N(R⁴)-,
X* un atome d'halogène,
Y, Z indépendamment l'un de l'autre un CH ou N, Y et Z n'étant pas simultanément CH,
R un atome d'hydrogène, un groupe alkyle (en C₁-C₁₂) ; alcényle (en C₂-C₁₀) ; alcynyle (en C₂-C₁₀); alkyle (en C₁-C₆) qui est substitué de 1 à 4 fois par des restes du groupe constitué d'un atome d'halogène, d'un groupe alcoxy (en C₁-C₄), alkylthio (en C₁-C₄), CN, [alcoxy (en C₁-C₄)]-carbonyle et alcényle (en C₂-C₆) ; ou cycloalkyle (en C₃-C₈), lequel est non substitué ou substitué par des restes du groupe constitué d'un groupe alkyle (en C₁-C₄), alcoxy (en C₁-C₄), alkylthio (en C₁-C₄) et d'un atome d'halogène ; cycloalcényle (en C₅-C₈) ; phényl-alkyle (en C₁-C₄), lequel est non substitué dans le reste phényle ou est substitué par un ou plusieurs restes du groupe constitué d'un atome d'halogène, d'un groupe alkyle (en C₁-C₄), alcoxy (en C₁-C₄), haloalkyle (en C₁-C₄), alkylthio (en C₁-C₄), [alcoxy (en C₁-C₄]-carbonyle, [alkyle (en C₁-C₄)]-carbonyloxy, carbonamide, [alkyle (en C₁-C₄)]-carbonylamino, [alkyle (en C₁-C₄)]-aminocarbonyle, di-[(alkyle (en C₁-C₄)]-aminocarbonyle et nitro ; ou un reste des formules A-1 à A-10 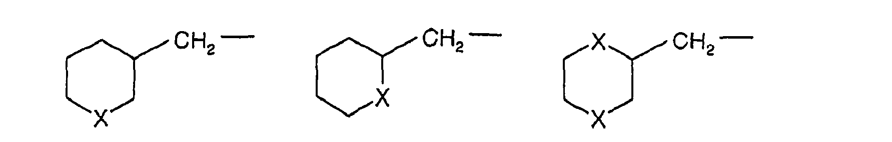 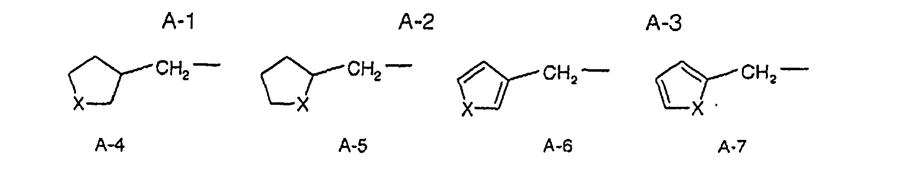 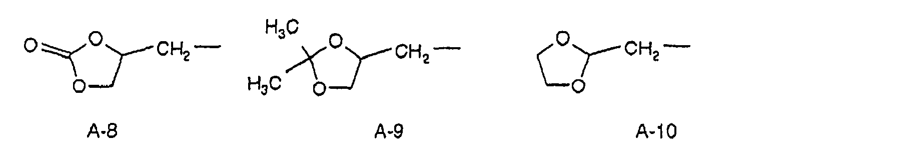 dans lesquelles
X est O, S, S(O) ou SO₂ ;
R¹ est un atome d'hydrogène ou un groupe alkyle (en C₁-C₃) ;
R² est un atome d'hydrogène, d'halogène, un groupe alkyle (en C₁-C₃), ou alcoxy (en C₁-C₃), chacun des deux derniers restes indiqués étant non substitué ou substitué une ou plusieurs fois par un atome d'halogène ou un groupe alcoxy (en C₁-C₃);
R³ est un atome d'hydrogène, d'halogène, un groupe alkyle (en C₁-C₃), alcoxy(en C₁-C₃) ou alkylthio(en C₁-C₃), chacun des trois restes indiqués précédemment n'étant pas substitué ou étant substitué une ou plusieurs fois par un atome d'halogène ou bien une ou deux fois par un groupe alcoxy (en C₁-C₃) ou alkylthio (en C₁-C₃) ; ou bien un reste de la formule NR⁵R⁶, cycloalkyle (en C₃-C₆), alcényle (en C₂-C₄), alcynyle (en C₂-C₄), alcényloxy (en C₃-C₄) ou alcinyloxy (en C₃-C₄) ;
R⁴ est un atome d'hydrogène, un groupe alkyle (en C₁-C₄) ou alcoxy (en C₁-C₄) et
R⁵ et R⁶ représentent indépendamment l'un de l'autre un atome d'hydrogène, un groupe alkyle (en C₁-C₄), alcényle (en C₃-C₄), haloalkyle (en C₁-C₄) ou alcoxy (en C₁-C₄),
**caractérisé en ce que**
a) on convertit un composé de la formule (II) 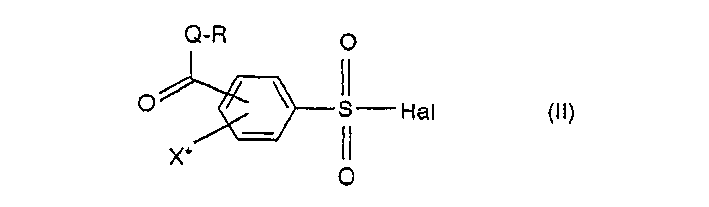 dans laquelle Hal est un atome d'halogène et R, Q et X* sont définis comme dans la formule (I),
par ammonolyse avec de l'ammoniac en un composé de la formule (III) 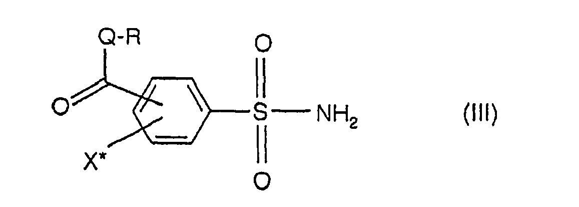 dans laquelle R, Q et X* sont définis comme dans la formule (I),
ou, de préférence,
(a1) on ammonolyse le composé de la formule (II) en le composé de la formule (III) et on réalise ce faisant la conversion dans un mélange de solvants organiques contenant
(1) un ou plusieurs hydrocarbures aromatiques éventuellement halogénés [solvant (1)] et
(2) un ou plusieurs solvants aprotiques polaires [solvant (2)], dans un rapport pondéral solvant (1) : solvant (2) de 20 : 1 à 1 : 1, de préférence de 10 : 1 à 1,4 : 1,
(b1) on convertit le composé (III) obtenu avec ou sans isolement intermédiaire en isocyanate de phénylsulfonyle de la formule (IV) avec du phosgène, 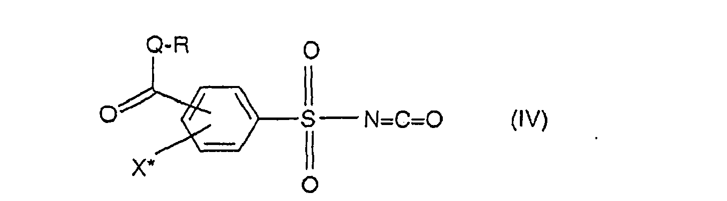 dans laquelle R, Q et X* sont définis comme dans la formule (I),
et on réalise ce faisant la conversion avec du phosgène dans un solvant organique en présence d'un isocyanate de la formule R'-NCO en tant que catalyseur, R' étant un reste hydrocarboné qui est non substitué ou substitué, ou bien d'un mélange de plusieurs de ces isocyanates en tant que catalyseur sans ajouter de base d'amine ou d'autres bases en tant que co-catalyseur,
et
(c) on convertit le composé (IV) obtenu avec ou sans isolement intermédiaire dans un solvant organique avec une amine de la formule (V) 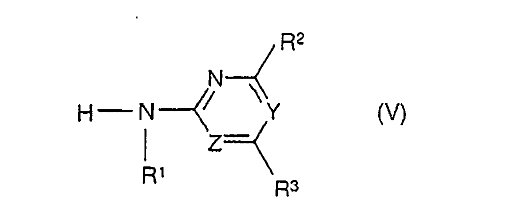 dans laquelle R¹, R², R³, Y et Z sont définis comme dans la formule (I), en sulfonylurée de la formule (I) ou ses sels,
ou de préférence
(c1) on convertit le composé (IV) obtenu avec ou sans isolement intermédiaire dans un solvant organique avec une amine de la formule (V) en composé de la formule (I) ou ses sels et on réalise ce faisant la conversion dans un mélange de solvants constitué d'un hydrocarbure aromatique éventuellement halogéné ayant un point d'ébullition supérieur à 110°C et d'un solvant aprotique polaire.

2. Procédé selon la revendication 1, **caractérisé en ce que** dans la formule (I) ou ses sels
Q représente un atome d'oxygène,
X* un atome d'halogène,
R un groupe alkyle (en C₁-C₄) ; alcényle (en C₂-C₄) ; alcinyle (en C₂-C₄) ; haloalkyle (en C₁-C₄) ou alcoxy (en C₁-C₄)-alkyle (en C₁-C₄),
R¹ un atome d'hydrogène,
R² un groupe alkyle (en C₁-C₄) ou alcoxy (en C₁-C₄),
R³ un groupe alkyle (en C₁-C₄) ou alcoxy (en C₁-C₄),
Y un atome d'azote ou un groupe de la formule CH et
Z un atome d'azote.

3. Procédé selon la revendication 2, **caractérisé en ce que** dans la formule (I) ou ses sels
X* représente un atome d'iode,
R un groupe méthyle ou éthyle,
R² un groupe méthoxy,
R³ un groupe méthyle,
Y un atome d'azote et
Z un atome d'azote.

4. Procédé selon l'une quelconque des revendications 1 à 3, **caractérisé en ce que** l'on réalise la conversion dans l'étape (a) selon la variante (a1) dans un mélange de solvants organiques contenant
(1) un ou plusieurs hydrocarbures aromatiques éventuellement halogénés du groupe constitué du xylène, du toluène, du chlorobenzène et du dichlorobenzène [solvant (1)] et
(2) un ou plusieurs solvants aprotiques polaires [solvant (2)] du groupe contenant des nitriles, des esters, des éthers, des amides, des cétones et des mélanges de deux ou plusieurs des solvants polaires.

5. Procédé selon l'une quelconque des revendications 1 à 4, **caractérisé en ce que** l'on réalise la conversion dans l'étape (b1) en présence d'un ou plusieurs isocyanates du groupe constitué d'isocyanates de N-alkyle, d'isocyanates de N-cycloalkyle et d'isocyanates de N-aryle en tant que catalyseur.

6. Procédé selon l'une quelconque des revendications 1 à 5, **caractérisé en ce que** l'on réalise la réaction dans l'étape (b1) en présence d'isocyanate de n-butyle ou d'isocyanate de cyclohexyle en tant que catalyseur.

7. Procédé selon l'une quelconque des revendications 1 à 6, **caractérisé en ce que** l'on réalise la conversion dans l'étape (c1) dans du xylène/acétate d'éthyle ou dans du xylène/acétonitrile.

8. Procédé pour la préparation de composés de la formule (IV) 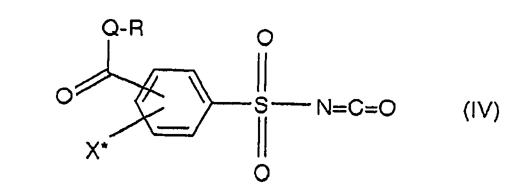 dans laquelle R et Q sont définis comme dans la formule (I) selon l'une quelconque des revendications 1 à 3, **caractérisé en ce que** l'on convertit un composé de la formule (III), 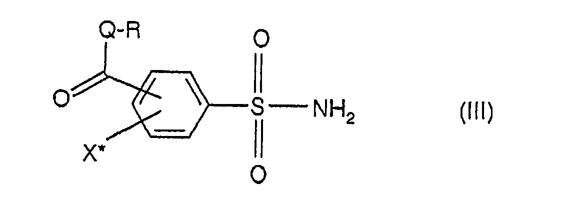 dans laquelle R, Q et X* sont définis comme dans la formule (IV),
avec du phosgène dans un solvant organique en présence d'un ou plusieurs isocyanates de la formule R'-NCO en tant que catalyseur, R' étant un reste hydrocarboné qui est non substitué ou substitué, sans ajouter de base d'amine ou d'autres bases en tant que co-catalyseur en isocyanate de phénylsulfonyle de la formule (IV).

9. Procédé selon la revendication 8, **caractérisé en ce que** l'on prépare pour la préparation du composé de la formule (IV) le composé de la formule (III) à utiliser, 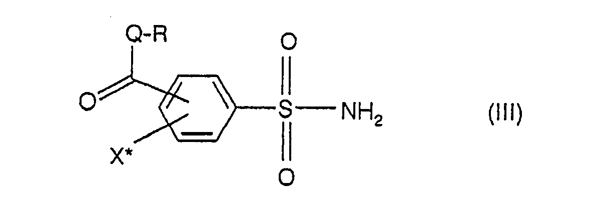 dans laquelle R, Q et X* sont comme définis dans la formule (IV), **en ce que** l'on convertit un composé de la formule (II) 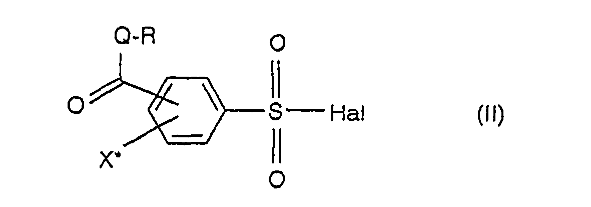 dans laquelle R, Q et X* sont comme définis dans la formule (III)
par ammonolyse avec de l'ammoniac en composé de la formule (III).

10. Procédé selon la revendication 9, **caractérisé en ce que** l'on réalise la réaction du composé de la formule (II) par ammonolyse dans un mélange de solvants organiques contenant
(1) un ou plusieurs hydrocarbures aromatiques éventuellement halogénés et
(2) un ou plusieurs solvants aprotiques polaires, dans un rapport pondéral solvant (1) : solvant (2) de 20 : 1 à 1 : 1, de préférence de 10 : 1 à 1,4 : 1.

11. Procédé selon la revendication 10, **caractérisé en ce que** l'on réalise la réaction du composé de la formule (II) par ammonolyse dans un mélange de solvants organiques contenant
(1) un ou plusieurs hydrocarbures aromatiques éventuellement halogénés choisis dans le groupe constitué du xylène, du toluène, du chlorobenzène et du dichlorobenzène, et
(2) un ou plusieurs solvants aprotiques polaires choisis dans le groupe contenant des nitriles, des esters, des éthers, des amides, des cétones et des mélanges de deux ou plusieurs des solvants polaires.

12. Procédé pour la préparation de composés de la formule (I) ou de ses sels, les composés étant définis selon les revendications 1 à 3, **caractérisé en ce que** l'on convertit
(b1) un composé de la formule (III), 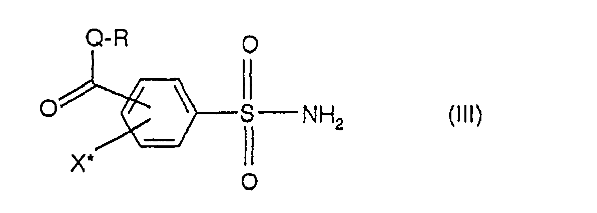 dans laquelle R, Q et X* sont définis comme dans la formule (I),
avec du phosgène en isocyanate de phénylsulfonyle de la formule (IV), 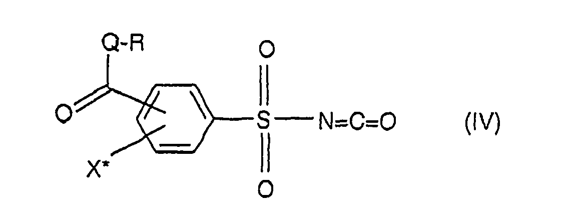 dans laquelle R, Q et X* sont comme définis dans la formule (I),
et on réalise ce faisant la conversion avec du phosgène dans un solvant organique en présence d'un isocyanate de la formule R'-NCO en tant que catalyseur, R' étant un reste hydrocarboné, lequel est non substitué ou substitué, ou un mélange de plusieurs de ces isocyanates en tant que catalyseur sans ajouter de base d'amine ou une autre base en tant que co-catalyseur, et
(c) on convertit le composé (IV) obtenu avec ou sans isolement intermédiaire dans un solvant organique avec une amine de la formule (V) 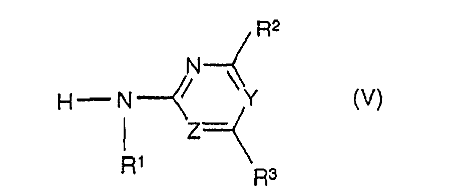 dans laquelle R¹, R², R³, Y et Z sont définis comme dans la formule (I), en sulfonylurée de la formule (I) ou ses sels,
ou de préférence
(c1) on convertit le composé (IV) obtenu avec ou sans isolement intermédiaire dans un solvant organique avec une amine de la formule (V) en composé de la formule (I) ou ses sels et on réalise ce faisant la conversion dans un mélange de solvants organiques constitué d'un hydrocarbure aromatique éventuellement halogéné avec un point d'ébullition supérieur à 110°C et d'un solvant aprotique polaire.

13. Procédé selon la revendication 12, **caractérisé en ce que** l'on réalise la conversion dans l'étape (b1) en présence d'un ou plusieurs isocyanates du groupe constitué d'isocyanates de N-alkyle, d'isocyanates de N-cycloalkyle et d'isocyanates de N-aryle en tant que catalyseur.

14. Procédé selon la revendication 12 ou 13, **caractérisé en ce que** l'on réalise la réaction dans l'étape (c1) dans du xylène/acétate d'éthyle ou dans du xylène/acétonitrile.

15. Procédé selon l'une quelconque des revendications 1 à 14, **caractérisé en ce que** sur le noyau phényle
- le groupe -CO-Q-R est en position ortho par rapport au groupe sulfonyle substitué et
- le groupe X* est en position para par rapport au groupe
- CO-Q-R.

16. Procédé selon l'une quelconque des revendications 1 à 15, **caractérisé en ce que** le reste X* sur le noyau phényle représente l'iode.
